(19) Europäisches Patentamt
European Patent Office
Office européen des brevets

(11) **EP 4 647 074 A1**

(12) **EUROPEAN PATENT APPLICATION**
published in accordance with Art. 153(4) EPC

(43) Date of publication:
**12.11.2025  Bulletin 2025/46**

(21) Application number: **24738509.9**

(22) Date of filing: **03.01.2024**

(51) International Patent Classification (IPC):
*A61K 31/519* (2006.01)     *A61K 9/70* (2006.01)
*A61P 25/18* (2006.01)

(86) International application number:
**PCT/CN2024/070458**

(87) International publication number:
**WO 2024/146576 (11.07.2024 Gazette 2024/28)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **04.01.2023  CN 202310010005**

(71) Applicant: **Novastage Pharmaceuticals
(Shenzhen), Ltd.
Shenzhen, Guangdong 518055 (CN)**

(72) Inventor: **TANG, Jiansheng
Shenzhen, Guangdong 518055 (CN)**

(74) Representative: **Cabinet Becker et Associés
25, rue Louis le Grand
75002 Paris (FR)**

(54) **TRANSDERMAL ADMINISTRATION SYSTEM FOR RISPERIDONE, AND PREPARATION METHOD THEREFOR AND USE THEREOF**

(57)    The present invention relates to a risperidone transdermal administration system. More specifically, the present invention relates to a transdermal administration system for continuously delivering risperidone or a pharmaceutically acceptable salt thereof at a therapeutically effective amount of blood drug concentration within 24 hours to 14 days, and a preparation method therefor and a use thereof.

*FIG. 4B*

**Description**

**Technical Field**

The present invention relates to a risperidone transdermal administration system. More specifically, the present invention relates to a transdermal administration system for continuously delivering risperidone or a pharmaceutically acceptable salt thereof at a therapeutically effective amount of blood drug concentration within 24 hours to 14 days, and a preparation method therefor and a use thereof.

**Background Art**

The transdermal route of administration is a route of administration that is superior to the oral route of administration, and maintains drug concentration in the blood at a constant level by continuously delivering drugs to the systemic blood system. The transdermal route of administration not only reduces the peak-to-trough fluctuations in drug concentration in the blood, but also avoids the first-pass effect. In addition, since the transdermal route of administration avoids direct contact between drugs and excipients and the gastrointestinal system, side effects such as nausea and vomiting that are often associated with the oral route of administration are significantly reduced or eliminated. Another advantage of the transdermal route of administration is that it is not affected by diet. Administration can be easily terminated when necessary by removing the transdermal patch from the skin. Furthermore, transdermal patches improve patient compliance by reducing the frequency of administration. In other words, patients may easily forget to take a tablet or capsule but may not forget to apply a transdermal patch. This is especially important for elderly patients and pediatric patients. For example, if the patch is designed to be worn 24 hours a day, the patient may easily remember to change the patch at 8 a.m. every morning. For example, if a patch is designed to be worn for 3.5 days, the patient may easily remember to change the patch every Monday at 8 a.m. and every Thursday at 8 p.m. For example, if the patch is designed to be worn for 7 days, the patient may easily remember to change the patch every Monday morning at 8 a.m. Transdermal patches are also clearly superior to injectables because application of transdermal patches is painless, whereas application of injectables is painful. Additionally, some injectable medications can only be used in hospitals.

Common dosage forms of transdermal route of administration include transdermal patch preparation. Currently common transdermal patch preparations include, but are not limited to, drug reservoir-type patches and matrix-type patches. Drug reservoir-type patch preparations are those in which the drug is contained in a reservoir with a drug-permeable base surface, and matrix-type patch formulations are those in which the drug is dissolved or dispersed in a polymeric matrix layer. Both types of designs also typically include a backing layer and a release film layer that is removed before use. In addition, patches often contain a penetration enhancer layer and an adhesive layer.

In recent years, the advantages of transdermal administration have enabled many drugs to be effectively administered via the transdermal route. These advances include the development of many physical methods to increase skin permeability and facilitate transdermal administration, e.g., using iontophoresis, electroporation, ultrasound, or microneedle. However, drugs that can be effectively and safely applied through the skin for 7 days or more without causing skin adhesion, skin irritation or sensitization are still limited.

The present invention relates to risperidone, the general formula of which is 3-[2-[4-{6-fluoro-1,2-benzisoxazol-3-yl)-1-piperidyl]ethyl]-6,7,8,9-tetrahydro-2-methyl-4H-pyrido[1,2-a]pyrimidin-4-one. The preparation method and pharmacological activity of this drug are described in US Patent No. 4,804,663 (corresponding to EP-0,196,132 (1984)).

This drug is currently widely used clinically as an antischizophrenia drug. Risperidone treats or prevents schizophrenia, mania, and dementia. Risperidone is a selective monoaminergic antagonist with unique properties. It has a high affinity for serotonergic 5-HT2 receptors and dopaminergic D2 receptors, and also binds to $\alpha$1-adrenergic receptors. It has a lower affinity for H1 histaminergic receptors and $\alpha$-2 adrenergic receptors, and can not bind to cholinergic receptors. In addition, risperidone treats or prevents the positive or negative symptoms of schizophrenia. Risperidone treats or prevents the positive or negative symptoms of schizophrenia, including hallucinations, delusions, and emotional withdrawal and blunted affect. In addition, it is characterized by relatively fewer extrapyramidal side effects (cold, stiffness, etc.) than traditional typical antipsychotics. Therefore risperidone is considered a very effective antischizophrenia drug that can greatly improve the quality of life of patients.

Risperidone is administered to patients orally as tablets, fine granules, and liquid preparations. However, oral administration has some disadvantages, such as susceptibility to first-pass effects in the liver after drug absorption and transient and undesirable high blood drug concentrations observed after administration. Furthermore, with oral administration, many side effects have been reported, such as gastrointestinal disorders, vomiting sensation, and loss of appetite. Furthermore, among patients with schizophrenia, it is believed that approximately 75% actually have difficulty taking oral preparations on a regular basis. Therefore, in order to solve these problems regarding oral administration and prepare a preparation that patients can take easily, safely and sustainably, a method of applying transdermal patch preparation is provided, which can solve the above-mentioned problems regarding oral administration, and has the advantages of reducing administration frequency, improving compliance, and making it easier for drug application or withdrawal. Therefore, transdermal administration patches are expected to be a useful route of administration.

In practice, to produce antipsychotic effects or to alleviate behavioral disturbances associated with neurodegenerative diseases, risperidone is usually administered as tablets, oral solution, or intramuscular injection solution. For a number of reasons, it would be desirable to administer risperidone in a non-invasive manner, specifically as a transdermal patch that would allow controlled release of the drug.

Risperidone is a highly effective drug with a relatively narrow therapeutic index. It may lead to adverse side effects in response to overdose, most notably extrapyramidal syndrome (EPS) and, to a lesser extent, hypotension (due to peripheral alpha-adrenergic activity). To produce antipsychotic effects in patients, the total daily dose of risperidone ranges from about 2 to about 8 mg. To alleviate behavioral disturbances associated with neurodegenerative diseases, the total daily dose is generally small, usually in the range of about 0.5 to about 2 mg. For interindividual variability and for additional drug therapy, dose titration in patients is required to obtain an effective dose.

Risperidone is metabolized to 9-hydroxyrisperidone, which has pharmacological properties and potency comparable to the parent drug risperidone, but has a longer elimination half-life. Risperidone is more rapidly distributed into brain tissue for elimination than its metabolite 9-hydroxyrisperidone.

Isoquinoline-type genetic polymorphisms play a unique role in the metabolism of risperidone. Based on metabolic rate, humans can be classified as poor, intermediate, or extensive metabolizers. The metabolic rate is defined as the ratio of the urinary recovery of isoquinoline to the urinary recovery of metabolite 4-hydroxyisoquinoline of isoquinoline within 8 hours after oral ingestion of 10 mg of isoquinoline. In the Easterners, more than 99% of the population can be phenotyped because extensive metabolizers and poor metabolizers are quite rare. However, within the Caucasian race, only approximately 90% of the population can be phenotyped as extensive or intermediate metabolizers. About 10% of the population has poor metabolism and insufficient levels of isoquinoline hydroxylase. The duration of action and peak plasma levels of the active agents (risperidone and 9-hydroxyrisperidone) are largely dependent on the rate of isoquinoline metabolism in human subjects receiving risperidone. More specifically, high transient peak levels of risperidone may be achieved in poor metabolizers when the total daily dose is administered at a single dose. This may cause undesirable side effects such as extrapyramidal syndrome (EPS) and hypotension (due to adrenergic effects). In general, the rapid distribution of risperidone in plasma and brain tissue indicates that the drug can be better administered periodically in divided doses, and it is best to administer risperidone continuously at a controlled rate to avoid the possibility of excessive peak levels (and side effects) while maintaining a clinically effective drug level. If risperidone could be administered transdermally, then a rate-controlled transdermal administration system would clearly provide a practical solution to the above problems. Furthermore, it is desirable that the transdermal administration system can deliver a drug at a substantially constant rate for at least about 24 hours while keeping the amount of unused and depleted drug in the system to a minimum. Given the pharmacological characteristics of risperidone, continuous delivery of therapeutic amounts of risperidone by a transdermal administration system that relies primarily on skin permeability to control the rate of drug penetration is not possible. There is a need to develop a transdermal administration system that controls the rate of drug penetration, which itself controls the maximum rate of drug delivery through the skin.

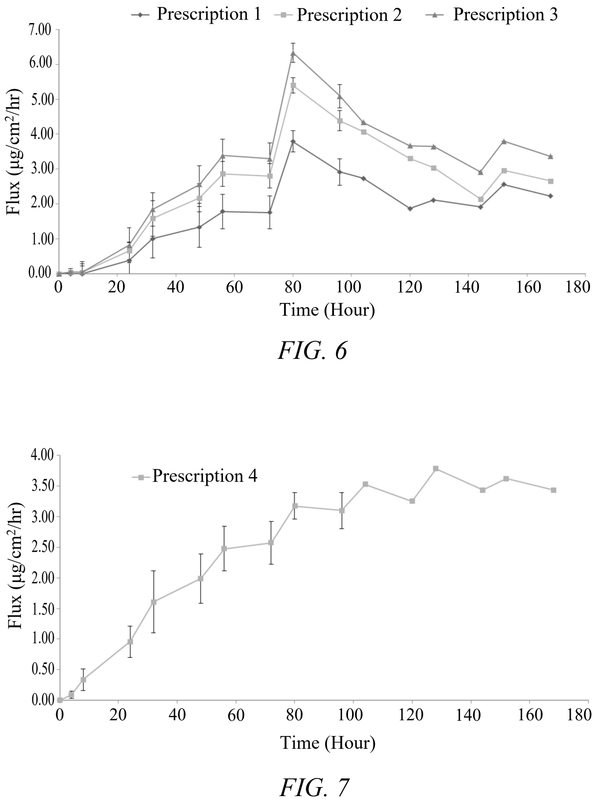

CN 101366705 B and US 8431152 B1 describe the preparation of corresponding patch preparations by dissolving a certain dose of risperidone in an organic solvent, and are not expected to contain risperidone dispersed in the matrix in a crystalline form. CN 101366705 B does not disclose that the patch can be applied continuously at a therapeutically effective dose and at a constant rate for 3 days (72 hours) or more, nor does it disclose that the patch contains risperidone dispersed in the matrix in a crystalline form. Example 4 and Fig. 6 of the aforementioned patent show that there is no linear relationship between the cumulative release rate and the increasing dosage of risperidone. According to the paper "Study on the Risperidone Transdermal Administration System" published by Chen Xiaojin, the inventor of the aforementioned patent, oleic acid is not the most preferred penetration enhancer for risperidone patch (see the last line on page 29), and azone, dodecanol and propylene glycol are the preferred penetration enhancers. However, azone has suspicious pharmacological activity or its safety is questionable. Dodecanol is a skin irritant. Propylene glycol is the solvent used to dissolve risperidone.

Therefore, there is an urgent need for a transdermal administration system of risperidone or a pharmaceutically acceptable salt thereof, which can deliver risperidone or a pharmaceutically acceptable salt thereof continuously at a therapeutically effective amount of blood drug concentration within 24 hours to 14 days, thereby improving the medication compliance of patients and providing long-term effective treatment for schizophrenia patients.

## Summary of the Invention

The present invention has found that it is difficult to achieve sustained drug release to a patient in a rate-controlled manner when administering a transdermal administration system containing risperidone, let alone to continuously deliver risperidone into the patient at a substantially constant rate within 24 hours to 14 days to maintain a therapeutically effective amount of blood drug concentration in the patient.

The present invention has now unexpectedly discovered that both lauryl lactate and long chain fatty alcohols are effective skin penetration enhancers for risperidone. They have no pharmacological activity and are non-irritating to the skin. The present invention has also unexpectedly found that microcrystalline risperidone without dissolution using solvents can provide long-term sustained transdermal delivery at a constant rate. When the transdermal administration system contains risperidone crystal form A and skin penetration enhancer (preferably lauryl lactate, long-chain fatty alcohol such as oleyl alcohol), it has good stability, and can continuously deliver drugs in a controlled manner at a constant rate within a continuous period of 3 days, 7 days, 10 days and 14 days, and the transdermal amount of risperidone is maintained above 65% of the maximum transdermal amount within day 3-day 7 after administration. The system continuously supplies risperidone to the skin, and the skin penetration enhancer allows the drug to penetrate the skin into the circulatory system at therapeutically effective doses.

The present invention also found that, the skin flux can be increased by controlling the dosage of the skin penetration enhancer and risperidone crystal form A, which is beneficial to achieving the above technical effects.

The present invention also found that, the skin flux can be increased by including undissolved risperidone crystal form A in the matrix layer of the risperidone administration system of the present invention, which is beneficial to achieving the above technical effects. The present invention also found that the viscosity of the transdermal administration system can be improved by adjusting the amounts of the pressure-sensitive adhesive and the cohesion promoter relative to the total weight of the matrix layer, so that the system can be well tolerated by the skin.

Penetration enhancers accelerate drug release from transdermal administration systems, which has the opposite effect to the purpose of sustained release. The present invention also found that a specific matrix combination can obtain a transdermal administration system of risperidone in both crystalline and amorphous states, which is beneficial to the sustained delivery of risperidone at a constant rate.

Therefore, the object of the present invention is to provide a matrix-type risperidone transdermal administration system that can continuously deliver risperidone or a pharmaceutically acceptable salt thereof at a constant rate within 24 hours to 14 days at a therapeutically effective amount of blood drug concentration.

Another object of the present invention is to provide a depot-type risperidone transdermal administration system that can continuously deliver risperidone or a pharmaceutically acceptable salt thereof within 24 hours to 14 days at a therapeutically effective amount of blood drug concentration.

Another object of the present invention is to provide a method for preparing a matrix-type risperidone transdermal administration system, which can continuously deliver risperidone or a pharmaceutically acceptable salt thereof within 24 hours to 14 days at a therapeutically effective amount of blood drug concentration.

Another object of the present invention is to provide the use of a therapeutically effective amount of a risperidone transdermal administration system in preparing a drug for treating or preventing schizophrenia, mania and dementia, which includes administering a therapeutically effective amount of a stable risperidone transdermal administration system to a subject in need.

Another object of the present invention is to provide the use of a therapeutically effective amount of a risperidone transdermal administration system in preparing a drug for treating or preventing positive and negative symptoms of schizophrenia, which includes administering a therapeutically effective amount of a stable risperidone transdermal administration system to a subject in need.

Another object of the present invention is to provide the use of a therapeutically effective amount of a stable risperidone transdermal administration system in preparing a drug for treating or preventing hallucinations, delusions, emotional withdrawal and emotional retardation symptoms of schizophrenia, which includes administering a therapeutically effective amount of a stable risperidone transdermal administration system to a subject in need.

Another object of the present invention is to provide a method of treating or preventing schizophrenia, mania and dementia, which includes administering a therapeutically effective amount of a stable risperidone transdermal administration system to a subject in need.

Another object of the present invention is to provide a method of treating or preventing positive and negative symptoms of schizophrenia, which includes administering a therapeutically effective amount of a stable risperidone transdermal administration system to a subject in need.

Another object of the present invention is to provide a method of treating or preventing hallucinations, delusions, emotional withdrawal and emotional retardation symptoms of schizophrenia, which includes administering a therapeutically effective amount of a stable risperidone transdermal administration system to a subject in need.

## Brief Description of the Drawings

Fig. 1 shows a schematic diagram of the backing layer, matrix layer and release layer in the three-layer matrix-type transdermal administration system of the present invention.

Fig. 2 shows a schematic diagram of the backing layer, matrix layer, skin-contacting adhesive layer and release layer in the four-layer matrix-type transdermal administration system of the present invention.

Fig. 3 shows a schematic diagram of the backing layer, matrix layer, semipermeable membrane or machine-woven fabric layer, skin-contacting adhesive layer and release layer in the five-layer matrix-type transdermal administration system of the present invention.

Figs. 4A-4F show schematic diagrams of matrix-type transdermal administration systems using overlapping adhesive films.

Fig. 5 shows the skin flux test results of comparative examples 1 to 6.

Fig. 6 shows the skin flux test results of examples 1 to 3.

Fig. 7 shows the skin flux test results of example 4.

Fig. 8 shows the XRPD spectrum of risperidone crystal form A.

Fig. 9 shows the XRPD spectrum of the transdermal patch preparation of crystalline risperidone.

Fig. 10 shows the skin flux test results of example 5 and example 8.

Fig. 11 shows the skin flux test results of example 9 and example 12.

Fig. 12 shows the skin flux test results of examples 26 to 33 and comparative example 7.

Fig. 13 show schematic diagrams of matrix-type transdermal administration systems using overlapping adhesive films in which the separation layer and the drug matrix layer/skin adhesive layer are cut into small pieces.

Fig. 14 shows a schematic view in which the separation layer and the drug matrix layer are not cut into small pieces.

Fig. 15 shows a schematic diagram in which the separation layer and the drug matrix layer are divided into 4 small pieces of 12.5 $cm^2$; There is one connection point between each small piece, and the intersecting black lines are the cut separation layer and drug matrix layer.

Fig. 16 shows a schematic diagram in which the separation layer and the drug matrix layer are divided into 4 small pieces of 12.5 $cm^2$; There is two connection points between each small piece, and the intersecting black lines are the cut separation layer and drug matrix layer.

Fig. 17 shows a schematic diagram in which the separation layer and the drug matrix layer are divided into 4 small pieces of 12.5 $cm^2$; There is no connection point between each small piece, and the intersecting black lines are the cut separation layer and drug matrix layer.

Fig. 18 shows the pieces without connection points of Fig. 17. When the inner patch (separation layer and matrix layer) moves forward, the 4 parts of the inner patch are separated from each other and arranged in disorder.

Fig. 19 shows the small pieces with two connection points shown in fig. 16. When the inner patch (separation layer and matrix layer) moves forward, the 4 small pieces of the inner patch are connected together in order.

Fig. 20 shows the skin flux test results of examples 42 to 45.

## **Detailed Description of Embodiments**

In one aspect, the present invention provides a risperidone transdermal administration system, which includes:

1) a backing layer;
2) a matrix layer, which contains risperidone dispersed in the matrix layer in crystalline and amorphous states, a skin penetration enhancer and a pressure-sensitive adhesive; and
3) a release layer.

In some embodiments, the crystalline form of risperidone in the risperidone transdermal administration system is crystalline form A, preferably micronized risperidone crystalline form A.

In some embodiments, the skin penetration enhancer is selected from one or more of $C_1$-$C_{30}$ fatty acids, fatty esters, or fatty alcohols;

preferably, the fatty acids are selected from $C_{12}$-$C_{22}$ unsaturated fatty acids, more preferably oleic acid;
preferably, the fatty esters are selected from esters produced by $C_1$-$C_{30}$ fatty acids and $C_1$-$C_{30}$ alcohols in combination, more preferably lauryl lactate;
preferably, the fatty alcohols are selected from $C_{13}$-$C_{30}$ alkenyl alcohols, more preferably oleyl alcohol.

In some embodiments, the matrix layer in the risperidone transdermal administration system includes the following components relative to the total weight of the matrix layer:

1) risperidone at a total amount of 9.5-15%, preferably 9.5-15% or 10-12%;
2) the skin penetration enhancer at an amount of 1-50%, preferably 2-30%, 8.5-30%, 8.5-25%, 8.5-15%; more preferably, the $C_1$-$C_{30}$ fatty acids at an amount of 8.5-15%, the fatty esters at an amount of 1-10%, and the fatty alcohols at an amount of 1-15%; and most preferably, oleic acid at an amount of 8.5-15%, lauryl lactate at an amount of 1-10%, 1-6% or 3-5%, $C_{13}$-$C_{30}$ alkenyl alcohol at an amount of 1-15%, 5-15% or 8-15%;

3) the pressure-sensitive adhesive at an amount of 50-82%, preferably 50-80% or 55-80%;

the total amount of all component in the matrix layer is 100%.

In some embodiments, the matrix layer in the risperidone transdermal administration system consists of the following components relative to the total weight of the matrix layer:

1) risperidone at a total amount of 9.5-15%, preferably 9.5-15% or 10-12%;

2) the skin penetration enhancer at an amount of 1-50%, preferably 2-30%, 8.5-30%, 8.5-25%, 8.5-15%; more preferably, the $C_1$-$C_{30}$ fatty acids at an amount of 8.5-15%, the fatty esters at an amount of 1-10%, and the fatty alcohols at an amount of 1-15%; and most preferably, oleic acid at an amount of 8.5-15%, lauryl lactate at an amount of 1-10%, 1-6% or 3-5%, $C_{13}$-$C_{30}$ alkenyl alcohol at an amount of 1-15%, 5-15% or 8-15%;

3) the pressure-sensitive adhesive at an amount of 50-82%, preferably 50-80% or 55-80%;

4) an antioxidant at an amount of 0%-1%, preferably 0.05%-0.5% or 0.1%-0.3%;

5) a cohesion promoter at an amount of 0-30%, preferably 1-5%;

6) a tackifier at an amount of 0-40%;

7) a plasticizer at an amount of 0-40%;

8) an additional penetration enhancer at an amount of 0-10%;

the total amount of all component in the matrix layer is 100%.

The additional penetration enhancer refers to another penetration enhancer different from 2).

In some embodiments, the pressure-sensitive adhesive is selected from one or more of acrylic adhesives, organosilicon adhesives, acrylic-organosilicon copolymer adhesives, polybutylene adhesives, styrene-isoprene-butylene copolymers, and styrene-butadiene-styrene copolymers.

In some embodiments, the cohesion promoter is selected from crospovidone, Eudragit E, Eudragit EPO, Eudragit S, Eudragit R. Plastoid B, and mixtures thereof. In some embodiments, the cohesion promoter is povidone K90, Eudragit EPO, and combinations of povidone K90 and Eudragit EPO, crospovidone CL-M and Eudragit EPO, povidone K90 and crospovidone CL-M and Eudragit EPO, povidone K90 and Plastoid B, crospovidone CL-M and Plastoid B, povidone K90 and crospovidone CL-M, the preferred combination is povidone K90 and Eudragit EPO.

In some embodiments, the antioxidant is selected from tocopherol, tocopheryl acetate, potassium metabisulfite, sodium metabisulfite, sodium bisulfite, sodium sulfite, propyl gallate, thioglycerol, sodium thiosulfate, sodium dioxide, sodium formaldehyde sulfoxylate, butylated hydroxytoluene (BHT); preferably, alpha-tocopherol (i.e. vitamin E), or a combination of alpha-tocopherol and butylated hydroxytoluene (BHT). In some embodiments, the further chelating agent as a synergistic antioxidant is selected from citric acid, tartaric acid, calcium disodium edetate, disodium edetate, and EDTA.

In some embodiments, the tackifier is selected from polybutenes, terpenes, and mixtures thereof.

In some embodiments, the plasticizer is selected from mineral oil, silicone oil, triethyl citrate, and mixtures thereof.

In some embodiments, the amount of the matrix layer in the risperidone transdermal administration system is from 30 g/m$^2$ to 700 g/m$^2$. In some embodiments, the amount of the matrix layer is 30 GSM to 700 GSM, 50 GSM to 700 GSM, 30 GSM to 100 GSM, 40 GSM to 150 GSM, 75 GSM to 150 GSM, 150 GSM to 300 GSM, or 350 GSM to 700 GSM. The specific amount of the matrix layer is selected from 30 GSM, 50 GSM, 55 GSM, 75 GSM, 100 GSM, 125 GSM, 150 GSM, 175 GSM or 200 GSM.

In some embodiments, during the preparation process of the risperidone transdermal administration system, risperidone is added in the form of micronized risperidone crystal form A, preferably the particle size of the micronized risperidone is less than or equal to 20 μm. In some embodiments, 90% or more of the particles have an average diameter of 20 μm or less, more preferably from 0.5 nm to 20 μm, and particularly preferably from 0.5 nm to 15 μm.

In some embodiments, aolubilizers or solvents are also used in the preparation of the matrix layer of risperidone transdermal administration system. Solubilizers or solvents have the effect of reducing viscosity (i.e. viscosity reducers). The solubilizer or solvent is selected from $C_1$-$C_6$ alkyl alcohol, n-heptane, ethyl acetate, toluene and mixtures thereof, preferably ethanol, isopropanol, n-heptane or ethyl acetate.

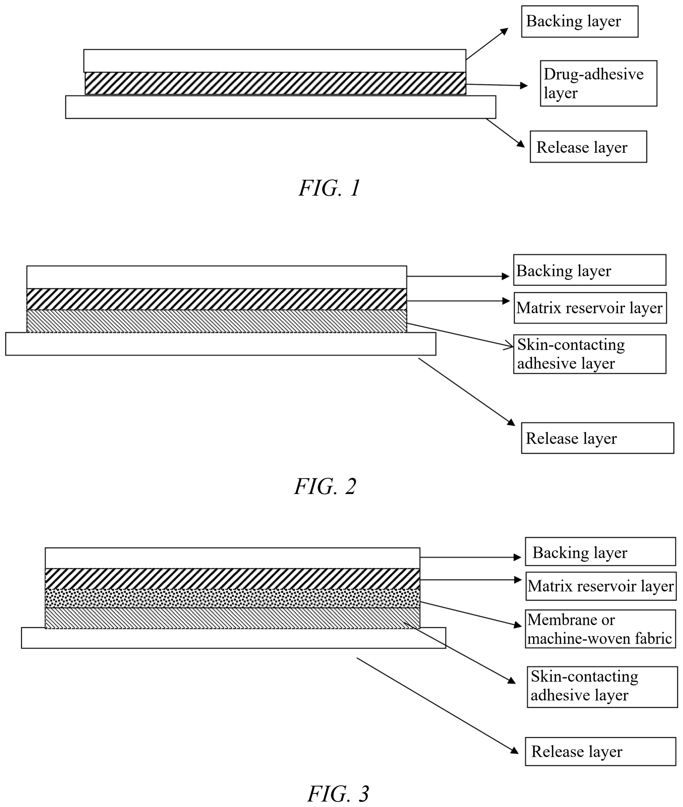

In some embodiments, a skin contact adhesive layer is added over the matrix layer (Fig. 2). The risperidone transdermal administration system includes:

1) a backing layer;

2) a matrix layer, which contains risperidone dispersed in the matrix layer in crystalline and amorphous states, a skin penetration enhancer and a pressure-sensitive adhesive;

3) a skin-contacting adhesive layer; and
4) a release layer.

In some embodiments, a rate control film layer (film or machine-woven fabric in Fig. 3) is added between the drug matrix reservoir layer and the skin contact adhesive layer. The risperidone transdermal administration system includes:

1) a backing layer;
2) a matrix layer, which contains risperidone dispersed in the matrix layer in crystalline and amorphous states, a skin penetration enhancer and a pressure-sensitive adhesive;
3) a semipermeable membrane or machine-woven fabric layer;
4) a skin-contacting adhesive layer; and
5) a release layer.

In some embodiments, the skin-contacting adhesive layer includes a pressure-sensitive adhesive and optionally risperidone and optionally other excipients selected from one or more of cohesion promoters, antioxidants, anti-skin irritation agents, tackifiers, plasticizers, solubilizers, solvents and desiccants.
In some embodiments, the risperidone transdermal administration system includes:

1) a backing layer;
2) an overlapping adhesive film layer, preferably the material of the overlapping adhesive film layer is selected from one or more of siloxane, polyisobutylene, or styrene-isoprene-styrene copolymer; preferably, the amount of the overlapping adhesive film layer is 50-110 GSM;
3) a separation layer;
4) a matrix layer, which contains risperidone dispersed in the matrix layer in crystalline and amorphous states, a skin penetration enhancer and a pressure-sensitive adhesive; and
5) a release layer.

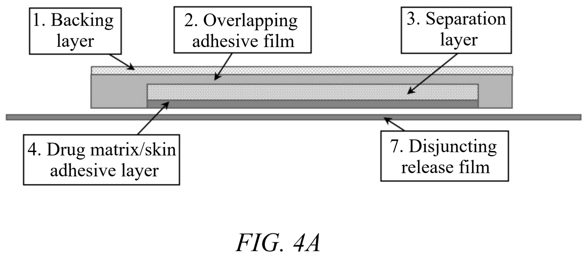

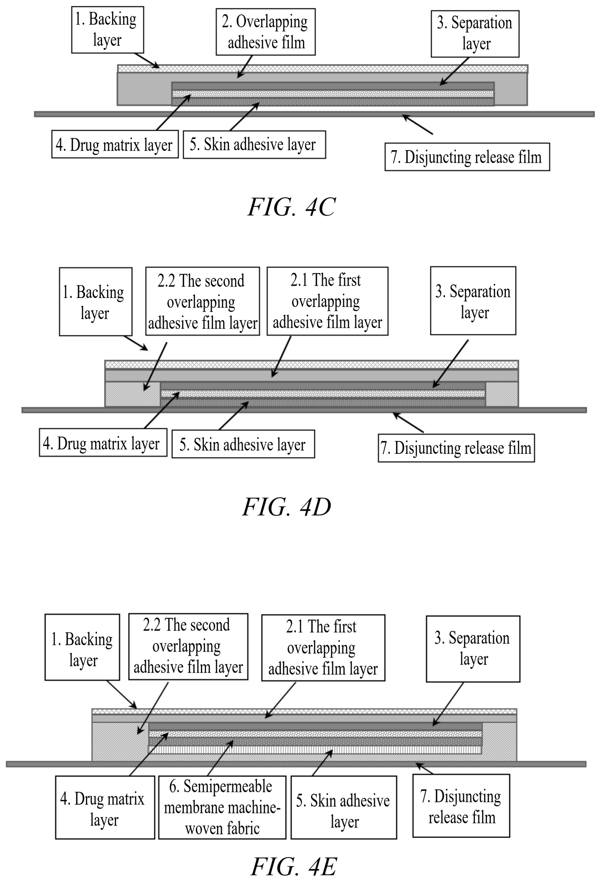

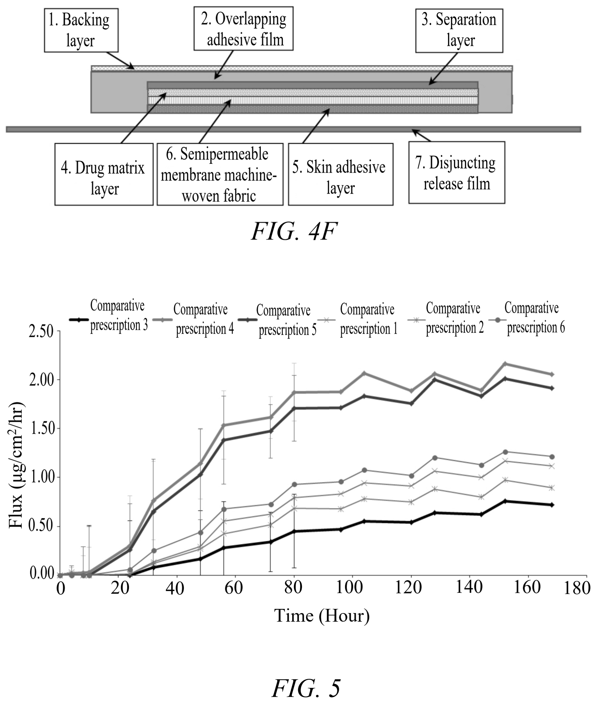

In some embodiments, the overlapping adhesive film layer of the risperidone transdermal administration system includes an adhesive, wherein the adhesive of the overlapping adhesive film layer is the same as or different from the adhesive of the drug matrix layer. Preferably, the solubility of a drug in the adhesive of the overlapping adhesive film layer is the same as or less than that of the drug in the adhesive of the matrix layer. More preferably, the solubility of the drug in the adhesive of the overlapping adhesive film layer is less than that of the drug in the adhesive of the matrix layer.
In some embodiments, the overlapping adhesive film layer can be one layer, two layers or multiple layers. Preferably, a first overlapping adhesive film layer close to the backing layer can prevent other overlapping adhesive film layers away from the backing layer from migrating to the backing layer or the side of the backing layer away from the skin.
In some embodiments, the adhesive of the first overlapping adhesive film layer is selected from Duro-Tak 387-2516, Duro-Tak 387-2287, Duro-Tak 387-4287, Duro-Tak 87-2051, Duro-Tak 87 -2052, Duro-Tak 87-2054, Duro-Tak 87-2852 or mixtures thereof. In some embodiments, the adhesive of the second overlapping adhesive film layer is selected from polyisobutylene, styrene-isoprene-styrene copolymer, dimethylsiloxane, or mixtures thereof.
In some embodiments, the risperidone transdermal administration system comprising overlapping adhesive film layers, sequentially from a backing layer to a disjuncting layer, includes: a backing layer, one or more overlapping adhesive film layers, a separation layer, a matrix layer, and a release layer (as shown in Fig. 4A); the backing layer, the one or more overlapping adhesive film layers and the release layer extend all around beyond the separation layer and the matrix layer; During application, the disjuncting layer is removed and the matrix layer in contact with the skin is sealed to the skin by one or more overlapping adhesive film layers.
In some embodiments, in the risperidone transdermal administration system comprising overlapping adhesive film layers, the overlapping adhesive film is one layer (as shown in Figs. 4A, 4C and 4F), and the risperidone transdermal administration system, sequentially from a backing layer to a disjuncting layer, includes:

i. a backing layer, an overlapping adhesive film layer, a separation layer, a matrix layer, and a release layer, and the backing layer, the overlapping adhesive film layer, and the release layer extend all around beyond the separation layer and the matrix layer; the structure illustrated in Fig. 4A is preferred; or
ii. a backing layer, an overlapping adhesive film layer, a separation layer, a matrix layer, a skin adhesive layer, and a release layer, and the backing layer, the overlapping adhesive film layer and the release layer extend all around beyond the separation layer, the matrix layer and the skin adhesive layer; the structure illustrated in Fig. 4C is preferred; or
iii. a backing layer, an overlapping adhesive film layer, a separation layer, a matrix layer, a semipermeable membrane layer, a skin adhesive layer, and a release layer; and the backing layer, the overlapping adhesive film layer and the release layer extend all around beyond the separation layer, the matrix layer, the semipermeable membrane layer and skin adhesive layer; the structure illustrated in Fig. 4F is preferred.

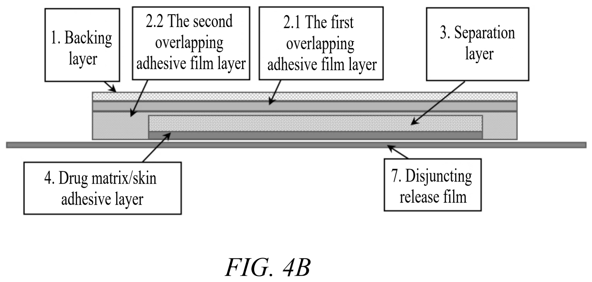

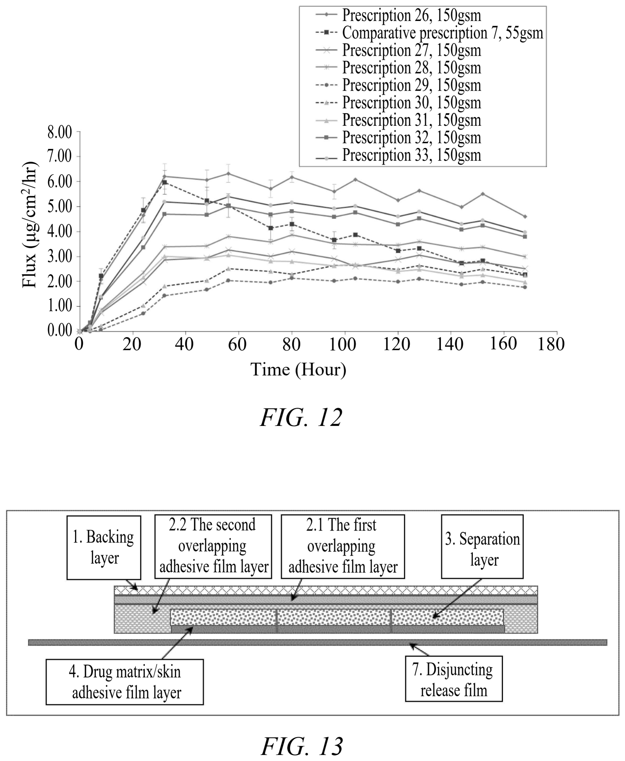

In some embodiments, in the risperidone transdermal administration system comprising overlapping adhesive film layers, the overlapping adhesive film is two layers (as shown in Figs. 4B, 4D, 4E and 13); During application, the disjuncting layer is removed and the matrix layer in contact with the skin is sealed to the skin by the second overlapping adhesive film layer; the risperidone transdermal administration system, from a backing layer to a disjuncting layer, includes:

i. a backing layer, a first overlapping adhesive film layer, a second overlapping adhesive film layer, a separation layer, a matrix layer, and a release layer, and the backing layer, the first overlapping adhesive film layer, the second overlapping adhesive film layer and the release layer extend all around beyond the separation layer and the matrix layer; the structure illustrated in Fig. 4B is preferred; or

ii. a backing layer, a first overlapping adhesive film layer, a separation layer, a matrix layer, a skin adhesive layer, a release layer, and a backing layer, and the first overlapping adhesive film layer and the release layer extend all around beyond the separation layer, the matrix layer and the skin adhesive layer, the second overlapping adhesive film layer is filled between the first overlapping adhesive film layer and the release layer, the structure illustrated in Fig. 4D is preferred; or

iii. a backing layer, a first overlapping adhesive film layer, a separation layer, a matrix layer, a semipermeable membrane layer, a skin adhesive layer, a second overlapping adhesive film layer, and a release layer, and the backing layer, the first overlapping adhesive film layer, the second overlapping adhesive film layer and the release layer extend all around beyond the separation layer, the matrix layer, the semipermeable membrane layer and the skin adhesive layer, and the second overlapping adhesive film layer extends toward the first overlapping adhesive film layer and wraps the separation layer, the matrix layer, the semipermeable membrane layer and the skin adhesive layer; the structure illustrated in Fig. 4E is preferred.

In some embodiments, the amount of the first overlapping adhesive film layer is 25-60 GSM; the amount of the second overlapping adhesive film layer is 25-60 GSM. preferably, the amount of the first overlapping adhesive film layer is 50-60 GSM; the amount of the second overlapping adhesive film layer is 50-60 GSM. In some specific embodiments, the amount of the first overlapping adhesive film layer is 25, 50, 55 or 60 GSM; The amount of the second overlapping adhesive film layer is 25, 50, 55 or 60 GSM.

In some embodiments, the backing layer is composed of a polymer elastic film, a polymer fabric, a bidirectional or multi-directional elastic nonwoven fabric, a stretchable polymer film, a stretchable machine-woven fabric, or a stretchable nonwoven fabric. In some embodiments, the backing layer is selected from polyester, polyethylene, polypropylene, polyvinyl chloride, polyethylene vinyl acetate, or polyurethane, preferably KOB051 and KOB053.

In some embodiments, the separation layer is selected from a flexible occlusive single-layer or multi-layer polymeric film. The polymer of the separation layer is selected from polyolefins, polyesters, polyethylene, polyvinylidene chloride or polyurethane. Preferably, the separation layer further includes an aluminum film.

In some embodiments, the separation layer includes a plurality of small pieces that are partially connected to each other, and the matrix layer includes a plurality of small pieces that are fully or partially connected. Preferably, the plurality of separation layer small pieces connected to each other in the separation layer are symmetrical or asymmetrical, the same or different. The plurality of small pieces connected to each other in the matrix layer are symmetrical or asymmetrical, the same or different. Further preferably, a part of the connection points of the separation layer and a part of the connection points of the matrix layer are all aligned or partially aligned.

In some embodiments, there are one or more connection points between adjacent pieces in the separation layer or matrix layer; 1, 2, 3, 4 or 5 connection points are preferred, and the length of each connection point is independently selected from the range of 0.5-3 mm.

In some embodiments, the overlapping separation layer pieces and the matrix layer pieces have the same shape, and the number of the pieces in each transdermal administration system unit is selected from 1 to 10.

In some embodiments, each small piece has an area of 1-25 cm$^2$, preferably 1 cm$^2$, 4 cm$^2$, 6 cm$^2$, 8 cm$^2$, 9 cm$^2$, 10 cm$^2$, 12.5 cm$^2$, 16 cm$^2$, or 25 cm$^2$.

In some embodiments, the matrix layer has a weight of 30 GSM to 700 GSM, 50 GSM to 700 GSM, 30 GSM to 100 GSM, 40 GSM to 150 GSM, 150 GSM to 300 GSM, or 350 GSM to 700 GSM.

On the other hand, the present invention provides a method of preparing a stable matrix-type risperidone transdermal administration system (as shown in Fig. 1), which includes the following steps:

step 1. mixing a skin penetration enhancer and optionally a cohesion promoter, an antioxidant, an anti-skin irritation agent, a tackifier, a plasticizer, a solubilizer, a solvent and a desiccant to obtain blend 1;

step 2. mixing the blend 1 and a pressure-sensitive adhesive to obtain blend 2;

step 3. adding micronized risperidone crystal form A or a pharmaceutically acceptable salt thereof to the blend 2 from step 2, and stirring the resulting mixture until risperidone or the pharmaceutically acceptable salt thereof is evenly suspended to obtain a wet drug mixture;

step 4. coating the wet drug mixture on a release layer;

step 5. drying to remove the solvent and the solubilizer to obtain a release layer/drug matrix layer laminated material; and

step 6. laminating the release layer/drug matrix layer laminated material to the backing layer to form a release layer/drug matrix layer/backing layer composite film.

In some embodiments, the present invention provides a method of preparing a matrix-type risperidone transdermal administration system (as shown in Fig. 2), comprising the following steps:

step 1. preparing a release layer/matrix layer/backing layer composite film according to the aforementioned steps 1 to 6;

step 2. preparing a solution or a suspension of a skin-contacting adhesive layer containing a pressure-sensitive adhesive and optionally risperidone crystal form A or a pharmaceutically acceptable salt thereof and optionally other excipients, coating same to a release layer and drying to form a release layer/skin-contacting adhesive layer laminated material; and

step 3. removing the release layer from the release layer/matrix layer/backing layer composite film, and laminating the adhesive layer side of the release layer/skin-contacting adhesive layer of step 1 to the matrix layer to form a release layer/skin-contacting adhesive layer/matrix layer/backing layer composite film.

In some embodiments, the present invention provides a method of preparing a matrix-type risperidone transdermal administration system (as shown in Fig. 3), comprising the following steps:

step 1. preparing a release layer/matrix layer/backing layer composite film according to the aforementioned steps 1 to 6 of the claims;

step 2. preparing a solution or a suspension of a skin-contacting adhesive layer containing a pressure-sensitive adhesive and optionally risperidone crystal form A or a pharmaceutically acceptable salt thereof and optionally other excipients, coating same to a release layer and drying to form a release layer/skin-contacting adhesive layer laminated material, and laminating the adhesive layer side to a semipermeable membrane or machine-woven fabric layer; and

step 3. removing the release layer from the release layer/matrix layer/backing layer composite film, and laminating the semipermeable membrane or machine-woven fabric layer side to the matrix layer to form a release layer/skin-contacting adhesive layer/semipermeable membrane or machine-woven fabric layer/matrix layer/backing layer composite film.

In some embodiments, the present invention provides a risperidone transdermal administration system (as shown in Fig. 4A), comprising the following steps:

step A: coating the adhesive of the overlapping adhesive film layer on the release film, and after drying, compounding same to the backing layer to obtain a backing layer/overlapping adhesive film/release film composite;

step B: preparing the release layer/drug matrix layer laminated material according to steps 1 to 5 of claim 32, and laminating the release layer/drug matrix layer laminated material to the separation layer to form a release layer/drug matrix layer/separation layer film;

step C: removing the release film of the backing layer/overlapping adhesive film/release film composite of step A, and applying the separation layer of the release layer/drug matrix layer/separation layer film of step B to the overlapping adhesive film;

step D: removing the release film from the drug matrix layer and applying an oversized release film on the drug matrix layer and the overlapping adhesive film layer;

step E: die-cuting the final patch so that the overlapping adhesive film layer extends beyond the separation layer and the drug adhesive layer in each direction.

In some embodiments, the present invention provides a risperidone transdermal administration system (as shown in Fig. 4C), comprising the following steps:

step A: coating the adhesive of the overlapping adhesive film layer on the release film, and after drying, compounding same to the backing layer to obtain a backing layer/overlapping adhesive film/release film composite;

step B: preparing a release layer/skin-contacting adhesive layer/drug matrix layer drug/separation layer composite film;

step C: removing the release film of the separation layer/overlapping adhesive film/release film composite of step A, and applying the separation layer of the release layer/skin-contacting adhesive layer/matrix layer/separation layer composite film formed in step B to the overlapping adhesive film;

step D: removing the release film from the skin-contacting adhesive layer and applying an oversized release film to the skin-contacting adhesive layer and the overlapping adhesive film layer;

step E: die-cutting the final patch so that the overlapping adhesive film layer extends beyond the separation layer, the matrix layer and the skin adhesive layer in each direction; preferably, the structure of Fig. 4C is formed.

In some embodiments, the present invention provides a risperidone transdermal administration system (as shown in Fig. 4F), comprising the following steps:

step A: coating the adhesive of the overlapping adhesive film layer on the release film, and after drying, compounding same to the backing layer to obtain a backing layer/overlapping adhesive film/release film composite;

step B: preparing the release layer/skin-contacting adhesive layer/semipermeable membrane or machine-woven fabric layer/drug matrix layer/separation layer composite film;

Step C: removing the release film of the backing layer/overlapping adhesive film/release film composite of step A, and applying the separation layer of the release layer/skin-contacting adhesive layer/semipermeable membrane or machine-woven fabric layer/drug matrix layer/separation layer composite film of step B to the overlapping adhesive film;

Step D: removing the release film from the skin-contacting adhesive layer and applying an oversized release film to the skin-contacting adhesive layer and the overlapping adhesive film layer;

step E: die-cutting the final patch so that the backing layer, the overlapping adhesive film layer and the release layer extend all around beyond the separation layer, the matrix layer, the semipermeable membrane or machine-woven fabric layer and the skin adhesive layer; preferably, the structure of Fig. 4F is formed.

In some embodiments, the present invention provides a risperidone transdermal administration system (as shown in Figs. 4B, 4D, 4E), comprising the following steps:

step A: coating the adhesive of the first overlapping adhesive film layer on the release film, and after drying, compounding same to the backing layer to obtain a backing layer/first overlapping adhesive film/release film composite film; coating the adhesive of the second overlapping adhesive film layer on the release film, and after drying, compounding same to the first overlapping adhesive film layer from which the release film is removed to obtain a backing layer/first overlapping adhesive film/second overlapping adhesive film/release film composite film.

step B: preparing a release layer/drug matrix layer/separation layer film (Fig. 4B), a release layer/skin adhesive layer/drug matrix layer/separation layer film (Fig. 4D), or a release layer/skin adhesive layer/semipermeable membrane machine-woven fabric layer/drug matrix layer/separation layer film (Fig. 4E);

step C: removing the release film of the backing layer/first overlapping adhesive film/second overlapping adhesive film/release film composite film of step A, and applying the separation layer of step B to the second overlapping adhesive film layer;

step D: removing the remaining release film of step C and applying an oversized release film on the side away from the backing layer;

step E: die-cutting the final patch so that the second overlapping adhesive film layer extends beyond the separation layer, the drug matrix layer, and optionally the skin adhesive layer and the semipermeable membrane machine-woven fabric layer in each direction; preferably, the structures of Figs. 4B, 4D, and 4E are obtained.

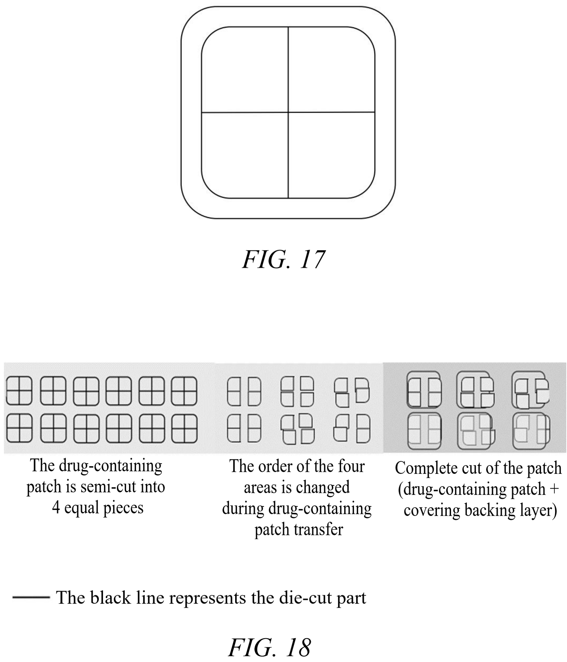

In some embodiments, after cutting the composite film prepared in the aforementioned B into a plurality of small pieces which are partially connected, the structure of the small pieces is obtained (as shown in Figs. 13 and 15-19); The structure in step C improves the arrangement order of the composite film in step B during transfer (as shown in Figs. 18-19).

In some embodiments, the wet drug mixture used to prepare the matrix layer contains undissolved risperidone crystal form A.

In some embodiments, the solvent in step 1 or step B is selected from $C_1$-$C_6$ alkyl alcohols, n-heptane, ethyl acetate, toluene, and mixtures thereof; preferably, ethanol, isopropanol, n-heptane or ethyl acetate.

In some embodiments, the risperidone transdermal administration system is packaged in a heat-sealed bag.

In another aspect, the present invention provides a use of a therapeutically effective amount of risperidone transdermal administration system in the preparation of a drug for treating or preventing schizophrenia, mania and dementia.

In another aspect, the present invention provides a use of a risperidone transdermal administration system in the preparation of a drug for treating or preventing positive and negative symptoms of schizophrenia. In some embodiments, the positive and negative symptoms of schizophrenia include hallucinations, delusions, and emotional withdrawal and blunted affect.

In another aspect, the present invention provides a method of treating or preventing schizophrenia, mania and dementia, which includes administering a therapeutically effective amount of a risperidone transdermal administration system to a subject in need.

In another aspect, the present invention provides a method of treating or preventing positive and negative symptoms of schizophrenia, which includes administering a therapeutically effective amount of a risperidone transdermal administration system to a subject in need. In some embodiments, the positive and negative symptoms of schizophrenia include hallucinations, delusions, and emotional withdrawal and blunted affect.

In some embodiments, the risperidone transdermal administration system is administered every 1 day, every 3 days, every 7 days, every 10 days, or every 14 days.

In some embodiments, the risperidone transdermal administration system continuously delivers risperidone or a pharmaceutically acceptable salt thereof at a therapeutically effective amount of blood drug concentration within 24 hours to 14 days.

In some embodiments, the risperidone transdermal administration system continuously delivers risperidone into the patient at a substantially constant rate within 24 hours to 14 days, preferably within 24 hours to 7 days.

In some embodiments, the maximum transdermal amount of risperidone occurs within 24-36 hours after administration of the risperidone transdermal administration system, and the transdermal amount of risperidone is maintained more than 65% of the maximum transdermal amount during day 3-day 7 after the administration; preferably, maintained 65%-90% of the maximum transdermal amount of risperidone; more preferably, maintained 75%-85% of the maximum transdermal amount of risperidone.

In some embodiments, for patches worn for 24 hours to 3 days, the matrix layer has a weight of 30 GSM (grams per square meter) to 100 GSM. For patches worn for 7 days, the matrix layer has a weight of 40 GSM to 150 GSM. For patches worn for 14 days, the matrix layer has a weight of 50 GSM to 700 GSM.

Definitions

As used herein, the term "pharmaceutically acceptable salt" refers to those salts that are suitable for contact with subjects (e.g., human subjects) within reasonable medical judgment without excessive toxicity, irritation, allergic reaction, etc., have a reasonable benefit/risk ratio, and are effective for their intended use.

The "pharmaceutically acceptable salt" mentioned in the present invention include inorganic acid addition salts and organic acid addition salts, can be prepared in situ during the final separation and purification of compounds, or can be prepared by reacting a purified compound in the form of a free base (for example, risperidone) with an appropriate organic acid or inorganic acid separately and separating the salts thus formed. Examples of inorganic acid addition salts include, but are not limited to, sulfates, pyrosulfate, bisulfate, sulfite, bisulfite, nitrate, phosphate, monohydrogen phosphate, dihydrogen phosphate, metaphosphate, pyrophosphate, hydrochloride, hydrobromide, hydroiodide, phosphite, borate, etc. Examples of organic acid addition salts include saturated or unsaturated $C_1$-$C_{30}$ fatty acid salts, including but not limited to monocarboxylic acid salts or dicarboxylic acid salts. Non-limiting examples include formate, glyoxylate, oxalate, acetate, glycolate, acrylate, pyruvate, malonate, propionate, 3-hydroxypropionate, lactate, glycerate, fumarate, maleate, oxaloacetate, crotonate, acetoacetate, 2-oxobutyrate, methylmalonate, succinate, malate, L-tartrate, DL-tartrate, meso-tartrate, dihydroxytartrate, butyrate, isobutyrate, hydroxybutyrate, levulinate, sorbate, itaconate, mesaconate, ketoglutarate, glutarate, succinate, methyl succinate, valerate, isovalerate, pivalate, cis-aconitate, trans-aconitate, ascorbate, citrate, isocitrate, adipate, caproate, benzoate, salicylate, gentisate, protocatechuate, gallate, cyclohexane carboxylate, pimelate, benzoate, chlorobenzoate, phthalate, isophthalate, terephthalate, terephthalate, phenyl acetate, toluate, o-toluate, m-toluate, p-toluate, dinitrobenzoate, benzenesulfonate, tosylate, citrate, mesylate, oleate, tosylate, methanesulfonate, naphthoate, glucoheptonate, lactobionate, lauryl sulfonate and isethionate, mandelate, homogentisate, suberate, caprylate, decanoate, laurate, palmitate, stearate, isostearate, oleate, elaidate, gangduosuanyan (刚多酸盐), erucate, nervonate, and ximenic acid salt (西门酸盐), hexadecatrienoic acid salt, linoleate, $\alpha$-linolenate, $\gamma$-linolenate, calendula acid salt, stearate, mead acid salt, eicosadienoic acid salt, eicosatrienoic acid salt, dihomo-$\gamma$-linolenate, arachidonic acid salt, docosadienoic acid salt, etc. and combinations thereof.

As used herein, the term "therapeutically effective amount" refers to the amount of a compound or molecule of the present invention that, when administered to a subject, (i) treats or prevents a specific disease, disorder or condition, (ii) attenuates, improves or eliminates one or more symptoms of the specific disease, disorder or condition, or (iii) prevents or delays the onset of one or more symptoms of the specific disease, disorder or condition described herein.

As used herein, the term "about" means plus or minus 10% of the indicated number. For example, "about 10%" may represent a range of 9% to 11%, and "about 1" may represent 0.9-1.1.

As used herein, the terms "optional" or "optionally" include both a selection and a non-selection. For example, "optional risperidone crystal form A" refers to the two situations of using risperidone crystal form A and not using risperidone crystal form A; "Optional other excipients" refers to two situations of using other excipients and not using other excipients.

As used herein, the term "treatment" refers to clinical intervention that attempts to alter the natural course of the individual being treated, and may be performed for prevention or in the course of clinical pathology. Desirable effects of treatment include, but are not limited to, preventing occurrence or recurrence of disease, alleviation of symptoms, weakening of any direct or indirect pathological consequences of the disease, preventing metastasis, decreasing the rate of disease progression, amelioration or alleviation of the disease state, and remission or improved prognosis.

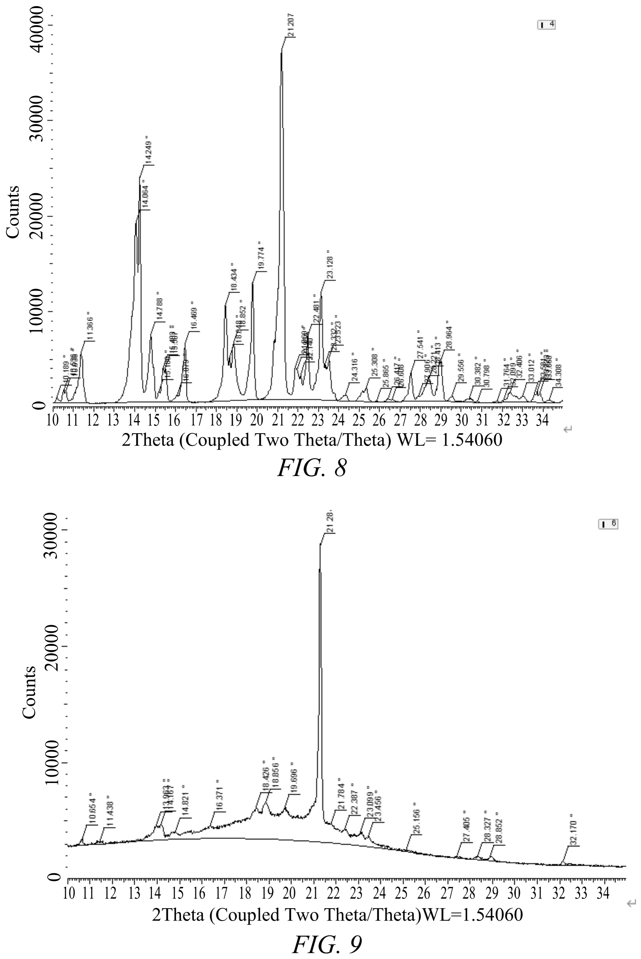

As used herein, the term "risperidone crystal form A" is described in WO 0212200A1, and its XRPD spectrum has characteristic peaks at 2θ values of $14.2 \pm 0.2°$, $21.3 \pm 0.2°$, and further has 2θ values of $10.6 \pm 0.2°$, $11.4 \pm 0.2°$, $16.4 \pm 0.2°$, $18.9 \pm 0.2°$, $19.9 \pm 0.2°$, $22.5 \pm 0.2°$, $23.3 \pm 0.2°$, $25.4 \pm 0.2°$, $27.6 \pm 0.2°$, $29.0 \pm 0.2°$. The risperidone crystal form A used in the present invention has an XRPD spectrum as shown in Fig. 8.

As used herein, the term "micronized" refers to particles having a particle size of 20 $\mu$m or less. In some embodiments, 90% or more of the particles have an average diameter of 20 $\mu$m or less, more preferably from 0.5 nm to 20 $\mu$m, and particularly preferably from 0.5 nm to 15 $\mu$m.

As used herein, the terms "backing layer" (in Figs. 1, 2, 3) and separation layer (in Figs. 4A-4F) serve as the upper surface of a transdermal patch and provide flexibility to the patch as main structural elements. Preferably, the backing layer is substantially impermeable to transdermally administered pharmaceutical compositions. The backing layer is preferably made from a sheet or film of flexible elastic material. The backing layer is preferably air-impermeable. The backing layer used in the patch of the present invention is preferably made of a flexible, biocompatible material that mimics the elastic properties of the skin and conforms to the skin during movement. Non-occlusive backing layers allow the area to breathe (i.e. promote water vapor transmission across the skin surface), whereas occlusive backing layers reduce air/vapor penetration. Preferably, the backing layer of the matrix-type transdermal administration systems (Figs. 1-4) is occlusive. Preferably, the backing layer contains synthetic polymers such as polyolefins, polyesters, polyethylene, polyvinylidene chloride, polypropylene, polyethylene vinyl acetate and polyurethanes. Preferably, the thickness of the backing layer is from about 0.5 mil to about 5 mil; more preferably, the thickness of the backing layer is from about 1 mil to about 3 mils. preferably, the oxygen transmission rate is from about 2 cc/m/24hr to about 100 cc/m/24hr, and more preferably, the oxygen transmission rate is from about 70 g/m/24hr to about 90 g/m/24hr. Preferably, the MVTR is from about 0.1 g/m/24hr to about 50 g/m/24hr, more preferably, the MVTR is from about 0.3 g/m/24hr to about 30 g/m/24hr. In a preferred embodiment, the backing layer is an of occlusive polyester film layer of approximately 2.0 mil thick (commercially available as Scotchpak 9733, Scotchpak 9735 and Scotchpak 9723, 3 M Drug Delivery Systems, St. Paul Minn.). Scotchpak 9733 composed of polyester and medium density polyethylene/ethylene vinyl acetate heat-sealing layer, and the laminate is translucent, conformable, occlusive and heat-sealable. It can be used in the matrix-type transdermal administration system shown in Figs. 1-3 and 4A-4F. More preferably, the backing layer includes a laminated material including an aluminum foil layer between polymer film layers, such as Scotchpak 9738 and Scotchpak 1109. When the patch is applied to the skin, the aluminum layer prevents light from coming into contact with the photosensitive risperidone.

As used herein, the term "separation layer" can be used to separate the overlapping adhesive film layer and the drug-containing matrix layer of the patch in Figs. 4A-4F. The separation layer is preferably a nonwoven polyester layer. Specific separation layers can be selected from Scotchpak1109, Scotchpak 9738, Scotchpak 9733 and Scotchpak 9754.

As used herein, the term "composite film" refers to a composite having a multi-layer structure. For example, in the present invention, "release layer/drug matrix layer/backing layer composite film" and "backing layer/first overlapping adhesive film/second overlapping adhesive film/release film composite film" all have multi-layer composite structures. Unless otherwise specified, "/" is used in the present invention to divide different layers in the composite film.

As used herein, the term "matrix layer" contains risperidone dispersed in the matrix layer in crystalline and amorphous states, a skin penetration enhancer and a pressure-sensitive adhesive and optional other excipients.

In addition, the matrix layer may also contain one or more other pharmaceutically acceptable excipients. Non-limiting examples of other excipients include, but are not limited to, cohesion promoters, antioxidants, anti-skin irritation agents, tackifiers, plasticizers, and the like.

Non-limiting examples of antioxidants include tocopherol, tocopheryl acetate, potassium metabisulfite, sodium metabisulfite, sodium bisulfite, sodium sulfite, propyl gallate, thioglycerol, sodium thiosulfate, sodium dioxide, sodium formaldehyde sulfoxylate, butylated hydroxytoluene (BHT), and chelating agents that act as synergistic antioxidants, including citric acid, tartaric acid, calcium disodium edetate, disodium edetate and EDTA. Preferably, the antioxidant is alpha-tocopherol (i.e. vitamin E), or a combination of alpha-tocopherol and butylated hydroxytoluene (BHT).

The pressure-sensitive adhesives include, but are not limited to, acrylic adhesives, organosilicon adhesives, acrylic-organosilicon copolymer adhesives, polybutylene adhesives, styrene-isoprene-butylene copolymers, styrene-butadiene-styrene copolymer, or a combination of two or more adhesives. Examples of acrylic adhesives are Duro-Tak® 387-2516, Duro-Tak®k 387-4280, Duro-Tak® 387-2287, Duro-Tak® 387-2510, Duro-Tak® 87-2196, Duro-Tak® 387-2051, Duro-Tak® 387-2052, Duro-Tak® 387-2054, Duro-Tak® 87-2194, Duro-Tak® 87-235A, Duro-Tak® 87-900A, Duro-Tak® 87-9301, Duro-Tak® 87- 4098, GEFVA GMS® 788, GEFVA GMS® 9073, Duro-Tak® 387-2353, Duro-Tak® 87-2074, Duro-Tak® 87-2852, Duro-Tak® 87-2054, GELVA®-73 Tak® 80-1196, Duro-Tak® 87-2070, Duro-Tak® 87-2979, Duro-Tak® 87-2888 and Duro-Tak® 87-2296 from Henkel Corporation (Bridgewater, New Jersey). Organosilicons BIO-PSA® 7-4401, BIO-PSA® 7-4402, BIO-PSA® 7-4501, BIO-PSA® 7-4502, BIO-PSA® 7-4601, BIO-PSA® 7-4602, SRS7-4502, SRS7-4501, SRS7-4502, SRS7-4602, BIO-PSA® 7-4101, BIO-PSA® 7-4102, BIO-PSA® 7-4103, BIO -PSA® 7-4201, BIO-PSA® 7-4202, BIO-PSA® 7-4203, BIO-PSA® 7-4301, BIO-PSA® 7-4302, BIO-PSA® 7-4302, BIO-PSA® 7-6101, BIO-PSA® 7-6102, BIO-PSA ® 7-6201, BIO-PSA® 7-6301, BIO-PSA® 7-6302 from Dupont (Midland, MI). Examples of polybutylene adhesives are combinations of two or more low molecular weight polyisobutylenes (Oppanol B10, Oppanol B11, and Oppanol B12 from BASF), medium molecular weight polyisobutylene (Oppnaol B50, Oppanol B80, and Oppnaol N80), and high molecular weight polyisobutylene (Oppanol B100, Oppanol B150, Oppanol N100, and Oppanol N150). An example of a styrene-isoprene-butylene copolymer is D1161 (Kraton).

Preferably, the pressure-sensitive adhesive is an acrylic adhesive, more preferably a hydroxyethyl functional acrylic copolymeric adhesive, such as Duro-Tak 387-2516, Duro-Tak 387-4280, 387 -2287, and Duro-Tak 387-2510. The amount of the adhesive is 50-82% relative to the total weight of the matrix layer; preferably, 50-80% or 55-80%. Preferably, the pressure-sensitive adhesive is a pressure-sensitive adhesive solution whose solvent is a poor solvent for risperidone. Examples of the solvents are n-heptane and ethyl acetate. n-Heptane is a poor solvent for risperidone, so most of the risperidone crystal form A is not dissolved in the pressure-sensitive adhesive solution, but exists in the pressure-sensitive adhesive blend as the original polymorph of the crystal. When the solvent is removed during the coating and drying process, risperidone is present in the dried drug matrix layer as the original stable polymorph A. Non-limiting examples of the tackifiers include polybutene, terpenes and mixtures thereof. The tackifier is present in the matrix layer in an amount from about 0% to about 30%, preferably from about 0% to about 25%, by weight of the adhesive material.

Non-limiting examples of the cohesion promoters are soluble polyvinylpyrrolidone (also known as povidone), insoluble crospovidone, Eudragit E, Eudragit EPO, Eudragit S, Eudragit R. Plastoid B, mixtures thereof, and the like. The matrix layer of the present invention contains a liquid skin penetration enhancer that causes cold flow around the patch, which can cause dark rings to appear on the skin during wear of the patch or transfer of adhesive to the skin when the patch is removed from the skin. Cohesion promoters increase the cohesion of the matrix layer, reducing cold flow, reducing the appearance of the dark ring on the skin or reducing the transfer of adhesive to the skin. An example of the soluble povidone is Povidone K90 and an example of the insoluble crospovidone is CL-M. The present inventors have surprisingly found that Povidone K90 is an excellent cohesion promoter. Preferred cohesion promotors are povidone K90, Eudragit EPO, and povidone K90 and Eudragit EPO, crospovidone CL-M and Eudragit EPO, povidone K90 and crospovidone CL-M and Eudragit EPO, povidone K90 and Plastoid B, crospovidone CL-M and Plastoid B, povidone K90 and crospovidone CL-M, and a preferred combination is one of povidone K90 and Eudragit EPO.

The skin penetration enhancers include, but are not limited to, $C_1$-$C_{30}$ fatty acids. In some embodiments this may specifically be $C_{7-30}$ fatty acid, $C_{7-22}$ fatty acid, or $C_{12-22}$ fatty acid. The $C_1$-$C_{30}$ fatty acids include saturated or unsaturated fatty acids, including but not limited to monocarboxylic acids or dicarboxylic acids. Non-limiting examples include formic acid, glyoxylic acid, oxalic acid, acetic acid, glycolic acid, acrylic acid, pyruvic acid, malonic acid, propionic acid, 3-hydroxypropionic acid, lactic acid, glyceric acid, fumaric acid, maleic acid, oxalacetic acid, crotonic acid, acetoacetic acid, 2-oxobutyric acid, methylmalonic acid, succinic acid, malic acid, L-tartaric acid, DL-tartaric acid, meso-tartaric acid, dihydroxytartaric acid, butyric acid, isobutyric acid, hydroxybutyric acid, levulinic acid, sorbic acid, itaconic acid, meconic acid, ketoglutaric acid, glutaric acid, methyl succinic acid, valeric acid, isovaleric acid, pivalic acid, cis-aconitic acid, trans-aconitic acid, ascorbic acid, citric acid, isocitric acid, adipic acid, caproic acid, benzoic acid, salicylic acid, gentisic acid, protocatechuic acid, gallic acid, cyclohexanecarboxylic acid, pimelic acid, phthalic acid, isophthalic acid, isophthalic acid, terephthalic acid, terephthalic acid, phenylacetic acid, toluic acid, o-toluic acid, m-toluic acid, p-toluic acid, mandelic acid, homogentisic acid, octanedioic acid, suberic acid, caprylic acid, capric acid, lauric acid, palmitic acid, stearic acid, isostearic acid, oleic acid, elaidic acid, gangduosuan (刚多酸), erucic acid, nervonic acid, and simonic acid (西门酸), hexadecatrienoic acid, Linoleic acid, α-linolenic acid, γ-linolenic acid, calendula acid, stearic acid, mead acid, eicosadienoic acid, eicosatrienoic acid, dihomo-γ-linolenic acid, arachidonic acid, docosadienoic acid and combinations thereof. Preferred is oleic acid, isostearic acid, or stearic acid.

The skin penetration enhancers also include fatty alcohols. The fatty alcohols include but are not limited to one or more saturated, monounsaturated or polyunsaturated $C_{7-30}$ alkyl alcohol or $C_{3-30}$ alkenyl alcohol, preferably one or more saturated, monounsaturated or polyunsaturated $C_{13-30}$ alkyl alcohol or $C_{13-30}$ alkenyl alcohol. This may include, but is not limited to, one or more of the following: tridecanol, myristyl alcohol, pentadecyl alcohol, cetyl alcohol, palmitoleyl alcohol, heptadecanol, stearyl alcohol, oleyl alcohol, linoleyl alcohol, nonadecanol, arachidonoyl alcohol, octyldodecanol, behenyl alcohol, sinapyl alcohol, lignoceryl alcohol and lignoceryl alcohol. Saturated fatty alcohol penetration enhancers may include but are not limited to one or more of the following: myristyl alcohol, isomyristyl alcohol, trans-isomyristyl alcohol, cetyl alcohol, isopalmityl alcohol, trans-isopalmityl alcohol, stearyl alcohol, isostearyl alcohol and trans-isostearyl alcohol. In some embodiments, the fatty alcohol is myristyl alcohol.

The skin penetration enhancers also include fatty esters. The fatty esters are esters produced by $C_1$-$C_{30}$ fatty acids and $C_1$-$C_{30}$ alcohols in combination. Non-limiting examples are lauryl lactate, myristyl lactate, cetyl lactate, palmitoyl lactate, Ceraphyl 31. ethyl laurate, methyl laurate, isopropyl myristate, isopropyl palmitate, Diisoadipate, medium chain fatty acid triglycerides, diethyl sebacate.

The skin penetration enhancers also include surfactants. Surfactants include, but are not limited to, one or more of glycerides (monoglycerides, diacylglycerol, and triglyceride), polyoxyethylene stearate, a mixture of trioctatetraenyl-4 phosphate, ethylene glycol palmityl stearate and diethylene glycol palmityl stearate, polyglycerol-3 diisostearate, PEG-6 stearate and ethylene glycol palmityl stearate and PEG-32 stearate, oleyl polyoxy-6 glyceride, lauroyl polyoxy-6 glyceride, capryloyl polyoxy-8 glyceride, propylene glycol monocaprylate, propylene glycol monolaurate, propylene glycol monolaurate, propylene glycol monocaprylate, Polyglyceryl-3 dioleate, a mixture of PEG-6 stearate and PEG-32 stearate, lecithin, cetyl alcohol, cholesterol, dioctyl sodium sulfosuccinate, sodium dodecyl sulfate, triethanolamine stearate, polyoxyethylene fatty alcohol ether, glyceryl monostearate, sorbitan monolaurate, lanolin alcohol and ethoxylated lanolin alcohol, sorbitan fatty acid ester, sucrose distearate. Span 20, Span 40, Span 80, Tween 20 Tween 40 Tween 80 pentadecanolide, glyceryl monolactate, glyceryl monostearate, glyceryl monooleate or combinations thereof.

According to a preferred embodiment of the present invention, the weight percentage of the skin penetration enhancer is 1-50% of the drug reservoir layer, preferably 2-30%.

Optional non-limiting solubilizers or solvents include $C_1$-$C_6$ alkyl alcohols, n-heptane, ethyl acetate, toluene, or a combination of two or more thereof. Solubilizers or solvents can also reduce the viscosity of the drug-adhesive suspension so that the suspension can be spread into a uniform film.

As used herein, the term "semipermeable membrane or machine-woven fabric layer" is used to contain a liquid or semi-solid matrix material within the matrix drug layer, and has a function of controlling the diffusion of risperidone from the liquid or semi-solid matrix drug layer to the skin-contacting adhesive layer. The semipermeable membrane includes, but is not limited to, ethylene-co-vinyl acetate copolymer membranes, polyethylene polymer membranes, and polypropylene polymer membranes. Non-limiting examples of ethylene-co-vinyl acetate copolymers include 3M Cotran 9702, Cotran 9712, Contan 9716 and Contran 9728. Non-limiting examples of polyethylene polymer membranes include Solupore. Non-limiting examples of polypropylene polymer membranes include Celgard 2400. The semipermeable membrane or machine-woven fabric layer and backing layer can be sealed together around the peripheral edge.

Suitable semipermeable membranes include continuous membranes and microporous membranes and can be made from woven or non-woven materials. The semipermeable membrane is preferably made of a flexible polymeric material commonly used by those skilled in the art. Polymer membranes that may be used to make the semipermeable membrane layer include, but are not limited to, those containing low density polyethylene, high density polyethylene, ethyl vinyl acetate copolymer, polypropylene, and other suitable polymers. In one embodiment, the semipermeable membrane layer is a microporous membrane prepared from an ethylene-vinyl acetate copolymer containing from about 0.5 to about 28 wt.% vinyl acetate. Suitable woven materials include Saatifil PES, such as PES 105/52 available from Saatitech, Inc. A suitable non-woven fabric is Sontara from DuPont Nonwovens Sontara Technologies. In a preferred embodiment, the semipermeable membrane layer is an ethylene-vinyl acetate copolymer membrane available from 3MTM, such as Cotran 9702, Cotran 9705, Cotran 9706, Cotran 9707, Cotran 9712, Cotran 9715, Cotran 9716 and Cotran 9728 (available from 3MTM). The thickness of the semipermeable membrane layer may generally be from about 10 um to about 100 um, preferably from about 15 um to about 50 um.

As used herein, the term "skin-contacting adhesive layer" serves to adhere the risperidone transdermal administration system to the skin surface. It can also be used to control the rate of risperidone delivery to the skin after the protective release layer is removed. The skin-contacting adhesive layer includes a pressure-sensitive adhesive and optional risperidone and optional other excipients selected from cohesion promoters, antioxidants, anti-skin irritation agents, tackifiers, plasticizers, solubilizers, and solvents. The pressure-sensitive adhesive includes one or more of acrylic adhesive, methacrylic adhesive, polyisobutylene adhesive, styrene-isoprene-styrene block copolymer adhesive, siloxane adhesive, acrylic-copolysiloxane copolymer adhesive; The acrylic adhesive is selected from Henkel's Duro-Tak adhesive 387-2051, 387-2054, 387-2353, 87-235A, 87-2852, 87-2074, 87-2677, 387-2516, 387-2287, 387-4287, 387-2510, crosslinked 387-2510, 87-900A, 87-9301, 87-4098, 87-2194, Gelva GMS788, Gelva GMS 9073, Gelva 737, Gelva 2655, Polythick 410-SA (Sanyo Chemical Industry Co., Ltd.); The polyisobutylene adhesive is selected from Oppanol N150, Oppanol B150, Oppanol N100, Oppnaol B100, Oppanol N80, Oppanol B80, Oppanol B10, B11, B12 and low molecular weight polybutylene H1900 from Ineos and mineral oil tackifier; The siloxane adhesive is selected from DuPont Bio-PSA 7-4100, 7-4200, 7-4300, 7-4400 and 7-4500, 7-4600 Bio-PSA SR7-4400, SRS7-4500, and SRS7-4600; The acrylic-copolysiloxane copolymer adhesive is selected from DuPont Bio-PSA 7-6100, 7-6200 and 7-6300. Acrylic adhesives combined with organosilicon adhesives and polyisobutylene combined with styrene-isoprene-styrene are also good adhesive choices.

As used herein, the term "overlapping adhesive film" refers to a patch comprising a backing layer-overlapping adhesive film-separation layer-matrix layer-release layer composed of an elastic material (as shown in Figs. 4A-4F, Fig. 13), which adheres to the skin more firmly than a patch comprising a backing layer-drug matrix layer-release layer (as shown in Fig. 1). The overlapping adhesive film can be one layer, two layers or multiple layers. The transdermal administration system of two layers of overlapping adhesive films is shown in Figs. 4B, 4D and 4E. The overlapping adhesive film layer 1 can prevent the overlapping adhesive film layer 2 from migrating to the side of the backing layer away from the skin. The overlapping adhesive films contain adhesives, examples of which include Duro-Tak 387-2516, Duro-Tak 387-2287, Duro-Tak 387-4287, Duro-Tak 87-2051, Duro-Tak 87-2052, Duro-Tak 87-2054 and other acrylates, siloxane, polyisobutylene, styrene-isoprene-styrene copolymer, or a combination of two or more adhesives. The present invention found that when the overlapping adhesive film is one or two acrylates, a considerable amount of nonvolatile risperidone migrates from the drug-containing matrix layer to the overlapping adhesive film layer, but when the overlapping adhesive film layer connected with the separation layer is siloxane, polyisobutylene or styrene-isoprene-styrene copolymer, only a small amount of nonvolatile risperidone migrates from the drug-containing matrix layer to the overlapping adhesive film layer.

As used herein, the term "release layer" includes, but is not limited to, silicone-coated polyester release liner available from many suppliers, fluoropolymer-coated polyester release liner from 3 M, and fluorosilicone-coated polyester release liner.

**The present invention has the advantages and technical effects as follows:**
The prior art does not disclose that a transdermal administration system containing risperidone or a pharmaceutically acceptable salt thereof can continuously release drugs to patients in a controlled manner at a constant rate, and not disclose a method for continuously delivering risperidone or a pharmaceutically acceptable salt thereof at a constant rate at a therapeutically effective amount of blood drug concentration within 24 hours to 14 days.

1. According to the invention, the risperidone crystal form A or a pharmaceutically acceptable salt thereof is combined with a skin penetration enhancer, so that the risperidone crystal form A has good stability, and the risperidone or a pharmaceutically acceptable salt thereof can be continuously delivered to permeate the skin into the blood circulation system. Unexpectedly, the transdermal administration system of the present invention enables sustained delivery of risperidone or a pharmaceutically acceptable salt thereof at a therapeutically effective amount of blood drug concentration within 24 hours to 14 days.

2. According to the invention, the skin flux can be increased by controlling the ratio of the skin penetration enhancer and risperidone crystal form A, which is beneficial to achieving the above technical effects.

3. According to the invention, the skin flux can be increased by including undissolved risperidone crystal form A in the matrix layer of the risperidone administration system of the present invention, which is beneficial to achieving the above technical effects.

4. According to the invention, the viscosity of the transdermal administration system can be improved by adjusting the amounts of the pressure-sensitive adhesive and the cohesion promoter relative to the total weight of the matrix layer, so that the system can be well tolerated by the skin.

Therefore, the present invention obtains a stable risperidone transdermal administration system, which can continuously release the drug to patients in a rate-controlled manner, and continuously deliver risperidone or a pharmaceutically acceptable salt thereof at a constant rate at a therapeutically effective amount of blood drug concentration within 24 hours to 14 days. The risperidone transdermal administration system of the present invention has stable properties, is safe and non-irritating, has good adhesion and stickiness to the skin, and has good skin tolerance.

5. The present invention increases the stickiness of the patch to the skin through the use of overlapping adhesive film layers.

6. The present invention uses a different adhesive from the central drug-containing layer (i.e. matrix layer) in the overlapping adhesive film, thereby greatly reducing the migration of non-volatile component drug molecules from the drug-containing layer to the overlapping adhesive film layer.

7. In the present invention, the separation layer and the drug-containing layer are cut into small pieces of 2-20 cm$^2$ to increase the stickiness of the drug-containing layer to the skin.

8. The separation layer pieces and drug-containing layer of the present invention are partially connected to each other. This makes the die-cutting process easier than that without the partial connection. Small pieces with no partially connected become messy during the die-cutting process. The small pieces of the separation drug layers that are partially connected to each other are neatly organized during the die-cutting process.

## Examples

In order to make the objectives, technical solutions and advantages of the embodiment of the present invention more clear, the technical solutions in the embodiment of the present invention will be described clearly and completely below. Apparently, the described embodiment is a module embodiment of the present invention, but not all of the embodiments. Elements and features described in one embodiment of the present invention may be combined with elements and features illustrated in one or more other embodiments. It should be noted that for purposes of clarity, representation and description of components and processes known to those of ordinary skill in the art that are not relevant to the present invention have been omitted from the description. Based on the embodiments of the present invention, all the other embodiments obtained by those of ordinary skill in the art without any inventive efforts should fall within the scope of protection of the present invention.

**In vitro skin flux experiment:**

In vitro penetration testing was performed using a vertical statically modified Franz cell. The receptor chamber has a volume of 7 ml, is filled with a buffer solution at pH 7.0, and has an effective skin permeability of 0.61 cm$^2$. The human cadaver skin was mounted on the receptor chamber with the dermis side facing the receptor chamber. The matrix layer was placed on the stratum corneum side of the human cadaver skin. The O-ring was placed on top of the skin. The donor chamber was secured on top of the receptor chamber. The Franz cell was placed in a 32°C incubator on a magnetic stir plate. At each prearranged time point, 2 ml of solution was taken, the remaining solution was poured off, and refilled with new receptor fluid. The receptor fluid was immediately analyzed for the amount of risperidone by HPLC.

**Comparative examples 1 to 6:**

K 90, oleic acid, EPO, DL-alpha tocopherol, 0.002% ascorbyl palmitate NF, 0.1% butylated hydroxytoluene and risperidone were dissolved in ethanol. Crospovidone was added and mixed for 24 hours. Duro-Tak 387-2516 was added and mixed well. The wet blend was coated on a release film, dried, and then laminated to a backing layer Scotchpak 1109. Comparative examples 5 to 6 were prepared in a similar manner to comparative example 1. An in vitro skin flux test was performed and the results are shown in Table 1 and Fig. 5. The in vitro skin flux was low. From comparative example 1 to comparative example 3, under 3% risperidone, when oleic acid increased from 10% to 12% to 17%, the in vitro skin flux decreased. This was because when the solubilizer oleic acid was increased, the drug concentration in the oleic acid decreased and became unsaturated. Comparative examples 4 to 6 also had the same trend.

**Table 1. Skin flux of comparative example 1 to comparative example 6**

| Time (hours) | Average value of skin from two donors (S365 & S376) | | | | | |
|---|---|---|---|---|---|---|
| | Comparative example 1 | Comparative example 2 | Comparative example 3 | Comparative example 4 | Comparative example 5 | Comparative example 6 |
| | Comparative preparation 1 | Comparative preparation 2 | Comparative preparation 3 | Comparative preparation 4 | Comparative preparation 5 | Comparative preparation 6 |
| | 3.0% risperidone, 12% K 90, 12% oleic acid, 0.5% EPO, 0.1% DL-alpha tocopherol, 0.002% ascorbyl palmitate NF, 0.1% butylhydroxytoluene, 10% crospovidone, 62.2980% Duro-Tak 387-2516 | 3.0% risperidone, 12% K 90, 15% oleic acid, 1.25% EPO, 0.1% DL-alpha tocopherol, 0.002% ascorbyl palmitate NF, 0.1% butylhydroxytoluene, 10% crospovidone, 58.5480% Duro-Tak 387-2516 | 3% risperidone, 12% K 90, 17% oleic acid, 1% EPO, 0.1% DL-alpha tocopherol, 0.002% ascorbyl palmitate NF, 0.1% butylhydroxytoluene, 10% crospovidone, 56.7980% Duro-Tak 387-2516 | 3.5% risperidone, 12% K 90, 10% oleic acid, 1% EPO, 0.1% DL-alpha tocopherol, 0.002% ascorbyl palmitate NF, 0.1% butylhydroxytoluene, 10% crospovidone, 63.2980% Duro-Tak 387-2516 | 3.5% risperidone, 12% K 90, 12% oleic acid, 1% EPO, 0.1% DL-alpha tocopherol, 0.002% ascorbyl palmitate NF, 0.1% butylhydroxytoluene, 10% crospovidone, 61.2980% Duro-Tak 387-2516 | 3.5% risperidone, 12% K 90, 15% oleic acid, 1.25% EPO, 0.1% DL-alpha tocopherol, 0.002% ascorbyl palmitate NF, 0.1% butylhydroxytoluene, 10% crospovidone, 58.0480% Duro-Tak 387-2516 |
| 0 | 0.000 | 0.000 | 0.000 | 0.000 | 0.000 | 0.000 |
| 4 | 0.015 | 0.031 | 0.018 | 0.019 | 0.000 | 0.000 |

(continued)

| Time (hours) | Average value of skin from two donors (S365 & S376) | | | | | |
| --- | --- | --- | --- | --- | --- | --- |
| | Comparative example 1 | Comparative example 2 | Comparative example 3 | Comparative example 4 | Comparative example 5 | Comparative example 6 |
| | Comparative preparation 1 | Comparative preparation 2 | Comparative preparation 3 | Comparative preparation 4 | Comparative preparation 5 | Comparative preparation 6 |
| | 3.0% risperidone, 12% K 90, 12% oleic acid, 0.5% EPO, 0.1% DL-alpha tocopherol, 0.002% ascorbyl palmitate NF, 0.1% butylhydroxytoluene, 10% crospovidone, 62.2980% Duro-Tak 387-2516 | 3.0% risperidone, 12% K 90, 15% oleic acid, 1.25% EPO, 0.1% DL-alpha tocopherol, 0.002% ascorbyl palmitate NF, 0.1% butylhydroxytoluene, 10% crospovidone, 58.5480% Duro-Tak 387-2516 | 3% risperidone, 12% K 90, 17% oleic acid, 1% EPO, 0.1% DL-alpha tocopherol, 0.002% ascorbyl palmitate NF, 0.1% butylhydroxytoluene, 10% crospovidone, 56.7980% Duro-Tak 387-2516 | 3.5% risperidone, 12% K 90, 10% oleic acid, 1% EPO, 0.1% DL-alpha tocopherol, 0.002% ascorbyl palmitate NF, 0.1% butylhydroxytoluene, 10% crospovidone, 63.2980% Duro-Tak 387-2516 | 3.5% risperidone, 12% K 90, 12% oleic acid, 1% EPO, 0.1% DL-alpha tocopherol, 0.002% ascorbyl palmitate NF, 0.1% butylhydroxytoluene, 10% crospovidone, 61.2980% Duro-Tak 387-2516 | 3.5% risperidone, 12% K 90, 15% oleic acid, 1.25% EPO, 0.1% DL-alpha tocopherol, 0.002% ascorbyl palmitate NF, 0.1% butylhydroxytoluene, 10% crospovidone, 58.0480% Duro-Tak 387-2516 |
| 8 | 0.000 | 0.000 | 0.000 | 0.024 | 0.000 | 0.000 |
| 10 | 0.000 | 0.000 | 0.000 | 0.032 | 0.000 | 0.000 |
| 24 | 0.013 | 0.011 | 0.000 | 0.304 | 0.257 | 0.059 |
| 32 | 0.136 | 0.121 | 0.080 | 0.764 | 0.653 | 0.253 |
| 48 | 0.293 | 0.270 | 0.166 | 1.142 | 1.031 | 0.439 |
| 56 | 0.553 | 0.425 | 0.281 | 1.535 | 1.381 | 0.679 |
| 72 | 0.623 | 0.515 | 0.340 | 1.613 | 1.474 | 0.727 |
| 80 | 0.792 | 0.684 | 0.451 | 1.873 | 1.709 | 0.931 |
| 96 | 0.832 | 0.678 | 0.468 | 1.878 | 1.711 | 0.958 |
| 104 | 0.944 | 0.783 | 0.553 | 2.068 | 1.834 | 1.076 |
| 120 | 0.915 | 0.748 | 0.540 | 1.888 | 1.755 | 1.021 |
| 128 | 1.065 | 0.882 | 0.641 | 2.063 | 2.002 | 1.204 |
| 144 | 1.000 | 0.801 | 0.622 | 1.891 | 1.833 | 1.128 |
| 152 | 1.169 | 0.975 | 0.758 | 2.162 | 2.010 | 1.265 |
| 168 | 1.117 | 0.895 | 0.718 | 2.057 | 1.913 | 1.216 |

**Example 1 and Example 3**

Examples 1 to 3 were prepared in a similar manner to comparative example 1. Some dissolved drugs recrystallized upon removal of the solvent. When the solubilizer oleic acid was fixed at 10%, the risperidone concentration increased from 4.1772% to 4.9964% to 5.7904%. An in vitro skin flux test was performed and the results are shown in Table 2 and Fig. 6. The in vitro skin flux increased. This was because the concentration of the drug in oleic acid increased. However, due to the low initial drug saturation and sub-saturation of the drug after 80 hours, the skin flux decreased after 80 hours.

**Table 2. Skin flux for examples 1 to 3**

| Time (hours) | Average value of skin from two donors | | |
|---|---|---|---|
| | Example 1 | Example 2 | Example 3 |
| | Preparation 1 | Preparation 2 | Preparation 3 |
| | 4.1772% risperidone, 10% oleic acid, 10% crospovidone, 0.1% DL-alpha tocopherol, 0.002% ascorbyl palmitate NF, 0.1% butylhydroxytoluene, 37.8104% Dow Corning 7-6101, 37.8104% Dow Corning 7-6301 | 4.9964% risperidone, 10% oleic acid, 10% crospovidone, 0.1% DL-alpha tocopherol, 0.002% ascorbyl palmitate NF, 0.1% butylhydroxytoluene, 37.4008% Dow Corning 7-6101, 37.4008% Dow Corning 7-6301 | 5.7904% risperidone, 10% oleic acid, 10% crospovidone, 0.1% DL-alpha tocopherol, 0.002% ascorbyl palmitate NF, 0.1% butylhydroxytoluene, 37.0038% Dow Corning 7-6101, 37.0038% Dow Corning 7-6301 |
| 0 | 0.000 | 0.000 | 0.000 |
| 4 | 0.000 | 0.046 | 0.051 |
| 8 | 0.000 | 0.043 | 0.048 |
| 24 | 0.378 | 0.660 | 0.819 |
| 32 | 1.004 | 1.585 | 1.846 |
| 48 | 1.338 | 2.172 | 2.559 |
| 56 | 1.784 | 2.862 | 3.387 |
| 72 | 1.755 | 2.806 | 3.300 |
| 80 | 3.794 | 5.398 | 6.337 |
| 96 | 2.916 | 4.386 | 5.089 |
| 104 | 2.738 | 4.075 | 4.337 |
| 120 | 1.869 | 3.306 | 3.669 |
| 128 | 2.113 | 3.037 | 3.650 |
| 144 | 1.913 | 2.144 | 2.919 |
| 152 | 2.561 | 2.963 | 3.802 |
| 168 | 2.226 | 2.662 | 3.363 |

**Example 4:**

Example 4 was prepared in a similar manner to comparative example 1. Some dissolved drugs recrystallize upon removal of the solvent. An in vitro skin flux test was performed, and the results are shown in Table 3. The in vitro skin flux was very high and did not decrease within 168 hours. This was because the saturation of the 7.5% concentration was very high.

**Table 3. Skin flux of example 4**

| Time (hours) | Average value of skin from two donors |
|---|---|
| | 7.5% risperidone, 10% oleic acid, 20% crospovidone CL-M, 0.0583% DL-alpha tocopherol, 0.0167% ascorbyl palmitate NF, 0.1% butylhydroxytoluene, 21.8138 % light mineral oil, 24.3068% Oppanol 12 Solution, 16.2045% Oppanol 100 |
| 0 | 0.000 |
| 4 | 0.093 |
| 8 | 0.338 |
| 24 | 0.957 |
| 32 | 1.604 |
| 48 | 1.987 |

(continued)

| Time (hours) | Average value of skin from two donors |
|---|---|
| | 7.5% risperidone, 10% oleic acid, 20% crospovidone CL-M, 0.0583% DL-alpha tocopherol, 0.0167% ascorbyl palmitate NF, 0.1% butylhydroxytoluene, 21.8138 % light mineral oil, 24.3068% Oppanol 12 Solution, 16.2045% Oppanol 100 |
| 56 | 2.476 |
| 72 | 2.572 |
| 80 | 3.171 |
| 96 | 3.097 |
| 104 | 3.522 |
| 120 | 3.247 |
| 128 | 3.779 |
| 144 | 3.433 |
| 152 | 3.617 |
| 168 | 3.429 |

**Example 5 to example 8**

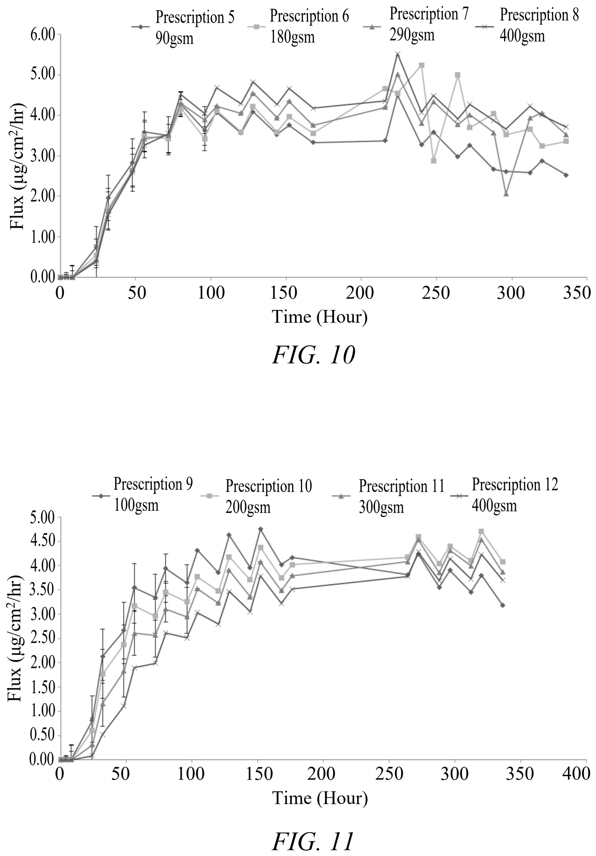

K 90, oleic acid, EPO, DL-alpha tocopherol, 0.002% ascorbyl palmitate NF, 0.1% butylated hydroxytoluene were dissolved in isopropanol. The solution was mixed for 24 hours. Duro-Tak 387-2516 was added and mixed well. Micronized risperidone crystal form A (Fig. 8, XRPD of API crystals) was added to form a homogeneous suspension in which undissolved risperidone crystals were suspended. The wet blend was coated on a release film, dried, and then laminated to a backing film Scotchpak 1109. Laminated material of 4 different coating weights (90 gsm, 180 gsm, 290 gsm and 400 gsm) were prepared. Microscopic analysis revealed that the laminated material was filled with risperidone crystals. The XRPD results (Fig. 9) indicate that the risperidone crystals have crystal form A. As shown in Table 4 and Fig. 10, the flux remains constant for 248 hours (10.5 days) with a coating weight of 90 gsm. When the coating weight was increased to 180 gsm, 290 gsm and 400 gsm respectively, the flux remains almost unchanged for 336 hours (14 days).

**Table 4. Skin flux of example 5 to example 8**

| time (hours) | Average value of skin from two donors | | | |
|---|---|---|---|---|
| | Example 5 | Example 6 | Example 7 | Example 8 |
| | Preparation 5 | Preparation 6 | Preparation 7 | Preparation 8 |
| | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 90gsm | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 180gsm | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 290gsm | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 400gsm |
| 0 | 0.000 | 0.000 | 0.000 | 0.000 |
| 4 | 0.000 | 0.011 | 0.030 | 0.000 |
| 8 | 0.000 | 0.000 | 0.000 | 0.000 |
| 24 | 0.748 | 0.555 | 0.444 | 0.395 |
| 32 | 1.968 | 1.699 | 1.637 | 1.548 |
| 48 | 2.839 | 2.638 | 2.653 | 2.600 |
| 56 | 3.584 | 3.474 | 3.417 | 3.261 |

(continued)

| time (hours) | Average value of skin from two donors | | | |
| --- | --- | --- | --- | --- |
| | Example 5 | Example 6 | Example 7 | Example 8 |
| | Preparation 5 | Preparation 6 | Preparation 7 | Preparation 8 |
| | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 90gsm | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 180gsm | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 290gsm | 12% risperidone, 10% oleic acid, 10% crospovidone, 0.1% DL-alpha tocopherol, 0.002% ascorbyl palmitate NF, 0.1% butylhydroxytoluene, 67.7980% Duro-Tak 387-2516, 400gsm |
| 72 | 3.497 | 3.426 | 3.514 | 3.546 |
| 80 | 4.274 | 4.176 | 4.293 | 4.524 |
| 96 | 3.633 | 3.422 | 3.886 | 4.041 |
| 104 | 4.075 | 4.116 | 4.226 | 4.682 |
| 120 | 3.561 | 3.586 | 4.051 | 4.284 |
| 128 | 4.087 | 4.217 | 4.546 | 4.822 |
| 144 | 3.528 | 3.586 | 3.938 | 4.272 |
| 152 | 3.754 | 3.964 | 4.341 | 4.658 |
| 168 | 3.329 | 3.556 | 3.755 | 4.174 |
| 216 | 3.375 | 2.879 | 4.193 | 4.350 |
| 224 | 4.500 | 4.993 | 5.014 | 5.513 |
| 240 | 3.269 | 3.698 | 3.807 | 4.070 |
| 248 | 3.587 | 4.044 | 4.337 | 4.486 |
| 264 | 2.972 | 3.522 | 3.772 | 3.913 |
| 272 | 3.264 | 3.661 | 4.010 | 4.270 |
| 288 | 2.669 | 3.243 | 3.569 | 3.877 |
| 296 | 2.618 | 3.359 | 2.062 | 3.657 |
| 312 | 2.585 | 3.226 | 3.935 | 4.226 |
| 320 | 2.883 | 3.622 | 4.059 | 4.009 |
| 336 | 2.523 | 3.162 | 3.519 | 3.711 |

**Example 9 to example 12**

K 90, oleic acid, EPO, DL-alpha tocopherol, 0.002% ascorbyl palmitate NF, 0.1% butylated hydroxytoluene were dissolved in isopropanol. The solution was mixed for 24 hours. Duro-Tak 387-2287 was added and mixed well. Micronized risperidone crystal form A was added to form a homogeneous suspension in which undissolved risperidone crystals are suspended. The wet blend was coated on a release film, dried, and then laminated to a backing film Scotchpak 1109. Laminated material of 4 different coating weights (90 gsm, 180 gsm, 290 gsm and 400 gsm) were prepared. Microscopic analysis showed that the laminated plate was filled with crystals. XRPD results indicate that the crystals have crystal form A. As shown in Table 4 and Fig. 11, the flux remains constant for 272 hours (11.5 days) with a coating weight of 100 gsm. When the coating weight was increased to 200 gsm, 300 gsm and 400 gsm respectively, the flux remains almost unchanged for 336 hours (14 days).

**Table 5 Skin flux of example 9 to example 12**

| time (hours) | Average value of skin from three donors | | | |
|---|---|---|---|---|
| | Example 9 | Example 10 | Example 11 | Example 12 |
| | Preparation 9 | Preparation 10 | Preparation 11 | Preparation 12 |
| | 12% risperidone, 10% K90, 10% oleic acid, 10% crospovidone, 1.25% EPO, 0.1% DL-alpha tocopherol, 0.002% ascorbyl palmitate NF, 0.1% butylhydroxytoluene, 56.5480% Duro-Tak387-2287, 100gsm | 12% risperidone, 10% K90, 10% oleic acid, 10% crospovidone, 1.25% EPO, 0.1% DL-alpha tocopherol, 0.002% ascorbyl palmitate NF, 0.1% butylhydroxytoluene, 56.5480% Duro-Tak 387-2287, 200gsm | 12% risperidone, 10% K90, 10% oleic acid, 10% crospovidone, 1.25% EPO, 0.1% DL-alpha tocopherol, 0.002% ascorbyl palmitate NF, 0.1% butylhydroxytoluene, 56.5480% Duro-Tak 387- 2287, 300gsm | 12% risperidone, 10% K90, 10% oleic acid, 10% crospovidone, 1.25% EPO, 0.1% DL-alpha tocopherol, 0.002% ascorbyl palmitate NF, 0.1% butylhydroxytoluene, 56.5480% Duro-Tak 387- 2287, 400gsm |
| 0 | 0.000 | 0.000 | 0.000 | 0.000 |
| 4 | 0.000 | 0.000 | 0.000 | 0.000 |
| 8 | 0.017 | 0.000 | 0.000 | 0.000 |
| 24 | 0.810 | 0.607 | 0.308 | 0.088 |
| 32 | 2.128 | 1.768 | 1.162 | 0.524 |
| 48 | 2.665 | 2.378 | 1.830 | 1.110 |
| 56 | 3.547 | 3.170 | 2.609 | 1.895 |
| 72 | 3.345 | 2.960 | 2.563 | 1.988 |
| 80 | 3.939 | 3.456 | 3.106 | 2.612 |
| 96 | 3.643 | 3.251 | 2.947 | 2.508 |
| 104 | 4.315 | 3.767 | 3.524 | 3.033 |
| 120 | 3.853 | 3.473 | 3.229 | 2.790 |
| 128 | 4.627 | 4.179 | 3.905 | 3.466 |
| 144 | 3.958 | 3.709 | 3.358 | 3.050 |
| 152 | 4.750 | 4.376 | 4.074 | 3.787 |
| 168 | 4.020 | 3.742 | 3.488 | 3.221 |
| 176 | 4.164 | 4.021 | 3.792 | 3.516 |
| 264 | 3.811 | 4.173 | 4.079 | 3.778 |
| 272 | 4.240 | 4.591 | 4.534 | 4.286 |
| 288 | 3.553 | 4.045 | 3.856 | 3.690 |
| 296 | 3.903 | 4.397 | 4.315 | 4.139 |
| 312 | 3.457 | 4.102 | 3.993 | 3.724 |
| 320 | 3.801 | 4.706 | 4.532 | 4.228 |
| 336 | 3.183 | 4.083 | 3.866 | 3.697 |

**Physical properties and skin adhesion - finger stickiness results**

The patch was placed on the workbench with the adhesive layer facing up. The adhesive was pressed with index finger for 5 seconds, then the index finger was lifted while holding part of the patch with the fingers of the other hand. The thumb was used to touch the front of the index finger to observe if there was any stickiness on the finger. If the adhesive was transferred to the index finger after the adhesive layer of the patch was lifted from the adhesive layer, the finger is sticky. The softer the adhesive, the less cohesive it is in terms of rheology, and the more adhesive transfer can be expected from the patch adhesive to the finger. Adhesive transfer to the skin can reduce skin adhesion and increase dark rings around the perimeter of the patch.

The results are shown in Table 6. The commercial patch was used as a reference for finger stickiness and was rated 10. It had no adhesive transfer to the fingers. In Example 13, preparation 13 had a finger stickiness of 15 and the adhesive transferred to 40% of the touching fingers. In examples 14 to 23, different levels of crospovidone CL-M, povidone K90 and Eudragit EPO were used to obtain preparations with finger stickiness in the range of 1 to 15 and no adhesive transfer to the contact fingers, except for preparation 22, because the amount of Eudragit was insufficient.

**Table 6. Finger stickiness results**

| Example# | Preparation# | Preparation composition | Finger stickiness | Amount of adhesive transferred to fingers | 168-hour skin adhesion score (0-4 scoring system) |
|---|---|---|---|---|---|
| 13 | Commercial patch | Commercial patch | 10 | 0 | Not carried out |
| 14 | 14 | 10% risperidone, 10% oleic acid, 0% crospovidone, 0.1% DL-alpha tocopherol, 0.002% ascorbyl palmitate NF, 0.1% butylhydroxytoluene, 79.7980% Duro-Tak 387-2516 | 15 | 40% | Not carried out |
| 15 | 15 | 10% risperidone, 10% oleic acid, 10% crospovidone, 0.1% DL-alpha tocopherol, 0.002% ascorbyl palmitate NF, 0.1% butylhydroxytoluene, 69.7980% Duro-Tak 387-2516 | 14 | 0 | 2 |
| 16 | 16 | 11% risperidone, 15% K90, 10% oleic acid, 0% EPO, 0.1% DL-alpha tocopherol, 0.002% ascorbyl palmitate NF, 0.1% butylhydroxytoluene, 63.7980% Duro-Tak 387-2287 | 13 | 0 | Not carried out |
| 17 | 17 | 11% risperidone, 5% K90, 10% oleic acid, 1.0% EPO, 0.1% DL-alpha tocopherol, 0.002% ascorbyl palmitate NF, 0.1% butylhydroxytoluene, 72.7980% Duro-Tak 387-2287 | 15 | 0 | 2 |
| 18 | 18 | 11% risperidone, 5% K90, 10% oleic acid, 1.5% EPO, 0.1% DL-alpha tocopherol, 0.002% ascorbyl palmitate NF, 0.1% butylhydroxytoluene, 72.2980% Duro-Tak 387-2287 | 14 | 0 | 2 |
| 19 | 19 | 11% risperidone, 10% K90, 10% oleic acid, 10% crospovidone, 0.5% EPO, 0.1% DL-alpha tocopherol, 0.002% ascorbyl palmitate NF, 0.1% butylhydroxytoluene, 58.2980% Duro-Tak 387-2287 | 9 | 0 | 1.5 |
| 20 | 20 | 11% risperidone, 10% K90, 10% oleic acid, 10% crospovidone, 0.75% EPO, 0.1% DL-alpha tocopherol, 0.002% ascorbyl palmitate NF, 0.1% butylhydroxytoluene, 58.0480% Duro-Tak 387-2287 | 9 | 0 | 2 |
| 21 | 21 | 11% risperidone, 10% oleic acid, 2.25% EPO, 0.1% DL-alpha tocopherol, 0.1% butylhydroxytoluene, 76.30% Duro-Tak 387- 2287 | 15 | 20% | Not carried out |

(continued)

| Example# | Preparation# | Preparation composition | Finger stickiness | Amount of adhesive transferred to fingers | 168-hour skin adhesion score (0-4 scoring system) |
|---|---|---|---|---|---|
| 22 | 22 | 11% risperidone, 10% oleic acid, 2.75% EPO, 0.1% DL-alpha tocopherol, 0.1% butylhydroxytoluene, 75.80% Duro-Tak 387- 2287 | 15 | 0 | 2 |
| 23 | 23 | 11% risperidone, 10% oleic acid, 1.5% EPO, 0.1% DL-alpha tocopherol, 0.1% butylhydroxytoluene, 77.3% Duro-Tak 387-2287 | 15 | 0 | 2 |

## Patch wearing test

One healthy volunteer participated in the patch wearing study. The skin of the left and right thighs was cleaned with wet paper towels and dried with dry paper towels. In each study, one or more patches were applied to the left and right thighs. After applying the patch, it was pressed with the palm for 30 seconds. The start date and time of the experiment were recorded. The adhesion score was recorded. The results are shown in Table 7, and the adhesion was scored using a five-point scale from 0 to 4.

Skin adhesion scores from the patch study are reported in Table 6. Most preparations have an adhesion score of 2 with no black rings caused by cold flow. The data show that the black rings caused by cold flow can be zeroed out by adjusting the amount of cohesion promoter. But its adhesion cannot meet the requirements.

**Table 7. Skin stickiness score**

| Degree of adhesion | Fraction |
|---|---|
| ≥ 90% (basically no lifting from the skin) | 0 |
| ≥ 75% to < 90% (only some edges lifting up from skin) | 1 |
| ≥ 50% to < 75% (less than half of the patch lifting from the skin) | 2 |
| > 0% to < 50% (more than half of the patch lifting the skin but not detaching) | 3 |
| 0% (the patch completely separates from the skin) | 4 |

## Examples 24 and 25

Each component was weighed according to Table 8. K 90, isostearic acid or stearic acid, EPO, DL-alpha tocopherol, and 0.1% butylated hydroxytoluene were dissolved in isopropanol. The solution was mixed for 24 hours. Duro-Tak 387-2287 was added and mixed well. Micronized risperidone crystal form A was added to form a homogeneous suspension in which undissolved risperidone crystals are suspended. The wet blend was coated on a release film, dried, and then laminated to a backing film Scotchpak 9738.

**Table 8**

| Example# | Preparation# | Preparation composition |
|---|---|---|
| 24 | 24 | 11% risperidone, 10% isostearic acid, 1.5% EPO, 0.1% DL-alpha tocopherol, 0.1% butylhydroxytoluene, 77.30% Duro-Tak 387- 2287 |
| 25 | 25 | 11% risperidone, 10% stearic acid, 1.5% EPO, 0.1% DL-alpha tocopherol, 0.1% butylhydroxytoluene, 77.30% Duro-Tak 387- 2287 |

## Example 26 Matrix-type transdermal administration system using overlapping adhesive films

Duro-Tak 387-2516 (10% w/w) and Duro-Tak 387-2287 (90% w/w) were mixed evenly, and then the mixture was coated on the release film, and the coated release film was dried (40°C 4 min, 85°C 4 min) and then compounded on the separation layer polyester fabric KOB053, with a coating thickness of 110 gsm, to prepare a backing layer/overlapping adhesive film/release film composite. The release film layer of the backing layer/overlapping adhesive film/release film composite was removed and the separation layer of the 10 cm$^2$ drug patch from example 23 was applied to the overlapping adhesive film. The release film was removed from the 10 cm$^2$ drug adhesive layer (drug matrix layer) and an oversized release film was applied on the drug adhesive layer and overlapping adhesive film layer. the final patch was die-cut so that the overlapping adhesive film layer extended 10 mm beyond the separation layer and the drug adhesive layer in each direction.

## Comparative example 7

Comparative prescription 7 patches were prepared according to example 4 of patent CN 101366705B. As shown in Table 9 and Fig. 12, the skin flux of comparative prescription 7 decreased rapidly after 48 hours, and the skin flux from 24 to 168 hours was not constant, but continuously decreasing. The skin flux at 168 hours was 61% lower than the maximum skin flux.

## Examples 26 to 33

Lauryl lactate, oleyl alcohol, EPO, DL-alpha tocopherol, 0.002% ascorbyl palmitate NF, 0.1% butylated hydroxytoluene were dissolved in isopropanol. The solution was mixed for 24 hours. Duro-Tak 387-2287 was added and mixed well. Micronized risperidone crystal form A was added to form a homogeneous suspension in which undissolved risperidone crystals are suspended. The wet blend was coated on a release film, dried, and then laminated to a backing layer film Scotchpak 1109. 5 laminated materials were prepared with a coating weight of 150 gsm. Microscopic analysis showed that the laminated plate was filled with crystals. As shown in Table 9 and Fig. 12, the skin flux from 24 hours to 168 hours was not only basically constant, but the transdermal amount at 168 hours was maintained at more than 70% of the maximum transdermal amount of risperidone.

## Table 9. Skin flux of examples 26 to 33 and comparative example 7

Average value of skin from three donors

Table 9-1

| time (hours) | Example 26 — Prescription 26: 11% risperidone, 20% lauryl lactate, 2.25% EPO, 0.1% DL-alpha tocopherol, 0.1% BHT, 66.5500% Duro-Tak 387-2287, 150gsm | Comparative example 7 — Comparative prescription 7: 9% risperidone, 6% azone, 8% propylene glycol, 5% 1-dodecanol, 72.0000% Duro-Tak 387-2287, 55gsm | Example 27 — Prescription 27: 11% risperidone, 12.5% lauryl lactate, 2% EPO, 0.1% DL-alpha tocopherol, 0.1% BHT, 74.3000% Duro-Tak 387-2287, 150gsm | Example 28 — Prescription 28: 11% risperidone, 15% lauryl lactate, 2% EPO, 0.1% DL-alpha tocopherol, 0.1% BHT, 71.8000% Duro-Tak 387-2287, 150gsm | Example 29 — Prescription 29: 11% risperidone, 10% oleyl alcohol, 1.25% EPO, 0.1% DL-alpha tocopherol, 0.1% BHT, 77.5500% Duro-Tak 387-2287, 150gsm |
|---|---|---|---|---|---|
| 0 | 0.000 | 0.000 | 0.000 | 0.000 | 0.000 |
| 4 | 0.262 | 0.309 | 0.144 | 0.171 | 0.000 |
| 8 | 2.076 | 2.222 | 0.746 | 0.858 | 0.071 |
| 24 | 4.642 | 4.848 | 1.983 | 2.359 | 0.726 |
| 32 | 6.206 | 5.970 | 2.864 | 3.398 | 1.442 |
| 48 | 6.054 | 5.237 | 2.939 | 3.419 | 1.670 |
| 56 | 6.320 | 5.036 | 3.271 | 3.804 | 2.035 |
| 72 | 5.718 | 4.133 | 3.013 | 3.582 | 1.961 |
| 80 | 6.181 | 4.301 | 3.206 | 3.867 | 2.139 |
| 96 | 5.600 | 3.654 | 2.915 | 3.512 | 2.019 |
| 104 | 6.083 | 3.875 | 2.608 | 3.479 | 2.112 |
| 120 | 5.245 | 3.232 | 2.884 | 3.450 | 1.988 |
| 128 | 5.626 | 3.326 | 3.061 | 3.595 | 2.096 |
| 144 | 4.975 | 2.737 | 2.720 | 3.306 | 1.874 |
| 152 | 5.504 | 2.833 | 2.767 | 3.370 | 1.975 |
| 168 | 4.602 | 2.277 | 2.523 | 2.996 | 1.769 |

| time (hours) | Example 26 | Comparative example 7 | Example 27 | Example 28 | Example 29 |
|---|---|---|---|---|---|
| | Prescription 26 | Comparative prescription 7 | Prescription 27 | Prescription 28 | Prescription 29 |
| | 11% risperidone, 20% lauryl lactate, 2.25% EPO, 0.1% DL-alpha tocopherol, 0.1% BHT, 66.5500% Duro-Tak 387-2287, 150gsm | 9% risperidone, 6% azone, 8% propylene glycol, 5% 1-dodecanol, 72.0000% Duro-Tak 387-2287, 55gsm | 11% risperidone, 12.5% lauryl lactate, 2% EPO, 0.1% DL-alpha tocopherol, 0.1% BHT, 74.3000% Duro-Tak 387-2287, 150gsm | 11% risperidone, 15% lauryl lactate, 2% EPO, 0.1% DL-alpha tocopherol, 0.1% BHT, 71.8000% Duro-Tak387-2287, 150gsm | 11% risperidone, 10% oleyl alcohol, 1.25% EPO, 0.1% DL-alpha tocopherol, 0.1% BHT, 77.5500% Duro-Tak 387-2287, 150gsm |
| Decline % | 27.18% | 61.86% | 22.87% | 22.51% | 17.32% |

Risperidone = risperidone, Eudragit EPO, DL-alpha tocopherol = DL-$\alpha$ tocopherol,
Lauryl lactate = lauryl lactate, oleyl alcohol = oleyl alcohol, Propylene glycol = propylene glycol, Azone = azone
1-Dodecanol=1-dodecanol,           gsm           =           grams           per           square           meter

$$\text{Decline \%} = (C_{max} - C_{168\,h})/C_{max} * 100$$

Table 9-2

| time (hours) | Example 30 | Example 31 | Example 32 | Example 33 |
|---|---|---|---|---|
| | Prescription 30 | Prescription 31 | Prescription 32 | Prescription 33 |
| | 11% risperidone, 10% lauryl lactate, 1.25% EPO, 0.1% DL-alpha tocopherol, 0.1% BHT, 77.5500% Duro-Tak 387 2287, 150gsm | 11% risperidone, 5% lauryl lactate, 1.5% EPO, 0.1% DL-alpha tocopherol, 0.1% BHT, 82.3000% Duro-Tak 387-2287, 150gsm | 11% risperidone, 5% lauryl lactate, 10.8% oleyl alcohol, 2.5% EPO, 0.1% DL-alpha tocopherol 1, 0.1% BHT, 70.5000% Duro-Tak 387-2287, 150gsm | 11% risperidone, 10% lauryl lactate, 10.8% oleyl alcohol, 2.75% EPO, 0.1% DL-alpha tocopherol, 0.1% BHT, 65.2500% Duro-Tak 387-2287, 150gsm |
| 0 | 0.000 | 0.000 | 0.000 | 0.000 |
| 4 | 0.072 | 0.187 | 0.360 | 0.191 |
| 8 | 0.234 | 0.821 | 1.360 | 1.380 |
| 24 | 1.037 | 2.163 | 3.358 | 3.739 |
| 32 | 1.823 | 3.005 | 4.687 | 5.184 |
| 48 | 2.045 | 2.922 | 4.656 | 5.090 |
| 56 | 2.520 | 3.052 | 5.014 | 5.395 |
| 72 | 2.403 | 2.824 | 4.674 | 5.051 |
| 80 | 2.295 | 2.795 | 4.806 | 5.155 |
| 96 | 2.650 | 2.622 | 4.583 | 4.918 |
| 104 | 2.654 | 2.687 | 4.751 | 5.007 |
| 120 | 2.488 | 2.398 | 4.284 | 4.591 |
| 128 | 2.644 | 2.483 | 4.513 | 4.793 |
| 144 | 2.347 | 2.214 | 4.071 | 4.301 |
| 152 | 2.497 | 2.260 | 4.233 | 4.438 |
| 168 | 2.242 | 1.980 | 3.781 | 3.956 |
| Decline % | 15.52% | 35.12% | 24.59% | 26.68% |

**Example 34** Matrix-type transdermal administration system using overlapping adhesive films

Duro-Tak 387-2051 and Duro-Tak 387-2054 were mixed evenly, and then the mixture was coated on the release film, and the coated release film was dried (40°C 4 min, 85°C 4 min) and then compounded on the backing layer KOB053 to prepare a backing layer/overlapping adhesive film/release film composite. The release film layer of the backing layer/overlapping adhesive film/release film composite was removed and the separation layer of the 10 cm$^2$ drug patch from example 23 was applied to the overlapping adhesive film. The release film was removed from the 10 cm$^2$ drug adhesive layer (drug matrix layer) and an oversized release film was applied on the drug adhesive layer and overlapping adhesive film layer. the final patch was die-cut so that the overlapping adhesive film layer extended 10 mm beyond the separation layer and the drug adhesive layer in each direction.

**Example 35** Matrix-type transdermal administration system using overlapping adhesive films

The matrix-type transdermal administration system of example 35 was prepared using a method similar to that of example 34. The backing layer KOB053/(Duro-Tak 387-2054 + Duro-Tak 387-2051) in example 34 was replaced with the backing layer KOB 053/(Duro-Tak 387-2516 + Duro-Tak 387-2287).

**Example 36** Matrix-type transdermal administration system using overlapping adhesive films

Duro-Tak 387-2516 (10% w/w) and Duro-Tak 387-2287 (90% w/w) were mixed evenly, and then the mixture was coated on the release film, and the coated release film was dried (40°C 4 min, 85°C 4 min) and then compounded on the backing layer KOB053, and the DURO-TAK 387-2516 and DURO-TAK 387-2287 mixed adhesive layer was covered with a polyisobutylene adhesive layer. A backing layer/overlapping adhesive film/release film composite was prepared. The release film layer of the backing layer/overlapping adhesive film/release film composite was removed and the separation layer of the 10 cm$^2$ drug patch from example 23 was applied to the overlapping adhesive film. The release film was removed from the 10 cm$^2$ drug adhesive layer (drug matrix layer) and an oversized release film was applied on the drug adhesive layer and overlapping adhesive film layer. the final patch was die-cut so that the overlapping adhesive film layer extended 10 mm beyond the separation layer and the drug adhesive layer in each direction.

**Example 37** Matrix-type transdermal administration system using overlapping adhesive films

The matrix-type transdermal administration system of example 37 was prepared using a method similar to that of example 36. The backing layer KOB(/Duro-Tak 387-2516 + Duro-Tak 387-2287)/PIB in example 36 was replaced with the backing layer KOB053/(Duro-Tak 387-2516+Duro-Tak 387-2287) /Bio-PSA 7-4301.

**Example 38** Matrix-type transdermal administration system using overlapping adhesive films

The matrix-type transdermal administration system of example 38 was prepared using a method similar to that of example 36. The backing layer KOB(/Duro-Tak 387-2516 + Duro-Tak 387-2287)/PIB in example 36 was replaced with the backing layer KOB053/(387-2516+387-2287)/SIS.

The test results of the migration of the non-volatile component risperidone from the central drug-containing layer to the overlapping adhesive film layer in examples 34-38 are summarized in Table 10.

Example 34: As shown in Table 10, when the adhesive of the overlapping adhesive film layer is a blend of two acrylic ester adhesives (Duro-Tak 387-2054, Duro-Tak 387-2051), a considerable amount of non-volatile risperidone migrates from the central drug-containing layer to the overlapping adhesive film layer.

Example 35: As shown in Table 10, when the adhesive of the overlapping adhesive film layer is a blend of two other acrylic ester adhesives (Duro-Tak 387-2516, Duro-Tak 387-2287), a considerable amount of non-volatile risperidone migrates from the central drug-containing layer to the overlapping adhesive film layer.

Example 36, example 37 and example 38 It was unexpectedly found that when the overlapping adhesive film layer in contact with the central backing layer was the siloxane Bio-PSA 7-4301, styrene-isoprene-styrene or polyisobutylene, there was little non-volatile risperidone migrates from the central drug-containing layer to the overlapping adhesive film layer.

**Table 10. Migration of the non-volatile component risperidone from the central drug-containing layer to the overlapping adhesive film layer**

| Time point | Risperidone content of covering adhesive layer | | | |
|---|---|---|---|---|
| | 60°C for 3 days | 60°C for 5 days | 60°C for 10 days | 40°C for one month |
| Example 34, overlapping adhesive film layer: backing layer KOB053/(Duro-Tak 387-2054 + Duro-Tak 387-2051) | 0.60% | 0.80% | 1.20% | 0.70% |
| Example 35, overlapping adhesive film layer: backing layer KOB 053/(Duro-Tak 387-2516 + Duro-Tak 387-2287) | 0.90% | 1.30% | 1.40% | 0.90% |
| Example 36, overlapping adhesive film layer: backing layer KOB(/Duro-Tak 387-2516 + Duro-Tak 387-2287)/PIB | 0.07% | 0.09% | 0.10% | 0.10% |
| Example 37, overlapping adhesive film layer: backing layer KOB053/(Duro-Tak 387-2516+Duro-Tak 387-2287)/Bio-PSA 7-4301 | 0.06% | 0.09% | 0.11% | 0.09% |
| Example 38, overlapping adhesive film layer: backing layer KOB053/(387-2516+387-2287)/SIS | 0.07% | 0.08% | 0.10% | 0.10% |
| PIB = polyisobutylene; SIS = styrene-isoprene-styrene copolymer | | | | |

**Comparative example 8** The separation layer and the drug matrix layer did not have a plurality of small pieces.

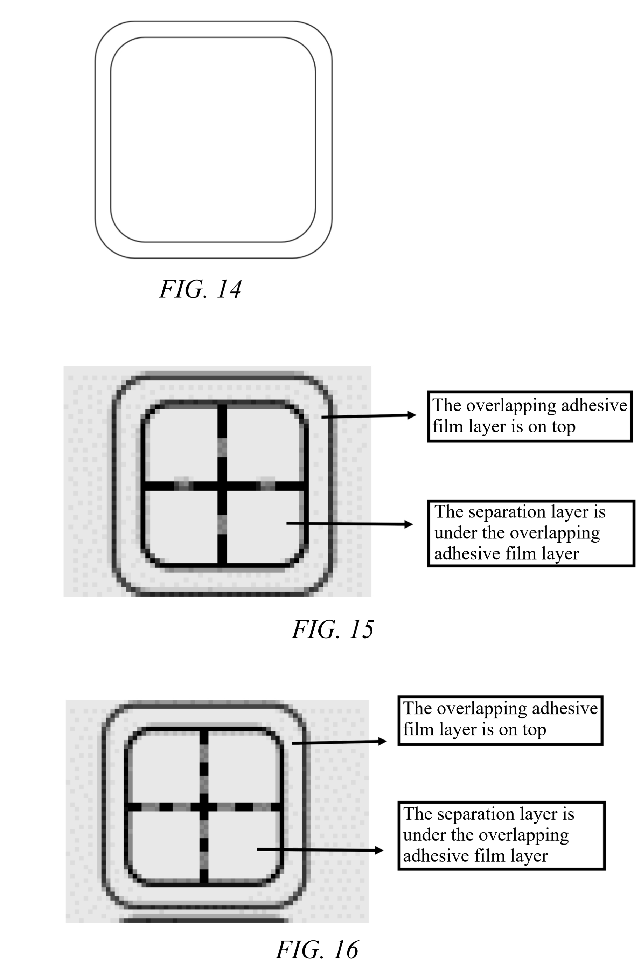

As shown in Fig. 14, a transdermal patch includes: (a) a backing layer of 82 cm$^2$ composed of an elastic material;

(b) an overlapping adhesive layer of 82 cm$^2$; (c) a separation layer of 50 cm$^2$; (d) a drug adhesion layer of 50 cm$^2$; (e) a release film. The separation layer and the drug matrix layer were not semi-cut into a plurality of small pieces. 15 volunteers completed 7 days of wear, and at the day 7 time point, the adhesion score for the edge of the overlapping layer was 0.1, but the adhesion score for the inner patch was 1.

**Example 39** A plurality of small pieces of the separation layer and the drug matrix layer had one connection point

Example 39 is as shown in Fig. 15, a transdermal patch includes: (a) a backing layer of 82 cm$^2$ composed of an elastic material; (b) an overlapping adhesive layer of 82 cm$^2$; (c) a separation layer of 50 cm$^2$; (d) a drug adhesion layer of 50 cm$^2$; (e) a release film. The separation layer and drug matrix layer were semi-cut into 4 small pieces. Among them, each small piece of the separation layer had one connection point. At the day 7 wear time point, the adhesion score for the edge of the overlapping layer was 0.1, but the adhesion score for the inner patch was 0.3.

**Example 40** A plurality of small pieces of the separation layer and the drug matrix layer had two connection points

As shown in Fig. 16, a transdermal patch includes: (a) a backing layer of 82 cm$^2$ composed of an elastic material; (b) an overlapping adhesive layer of 82 cm$^2$; (c) a separation layer of 50 cm$^2$; (d) a drug adhesion layer of 50 cm$^2$; (e) a release film. The separation layer and drug matrix layer were semi-cut into 4 small pieces. Among them, each small piece of the separation layer had two connection points. At the day 7 wear time point, the adhesion score for the edge of the overlapping layer was 0.1, but the adhesion score for the inner patch was 0.3.

**Comparative example 9** Small pieces without connection points were arranged out of order during transfer

As shown in Fig. 18, the separation layer and drug matrix layer are semi-cut into 4 pieces of 12.5 cm2 on a die-cutting machine. There are no connection points between small pieces (grey); The intersecting black lines are the cut separation layer and drug matrix layer. When the inner patch (separation layer and matrix layer) moves forward, the 4 parts of the inner patch are separated from each other and arranged in disorder.

**Example 41** Small pieces with two connection points achieved orderly arrangement during transfer

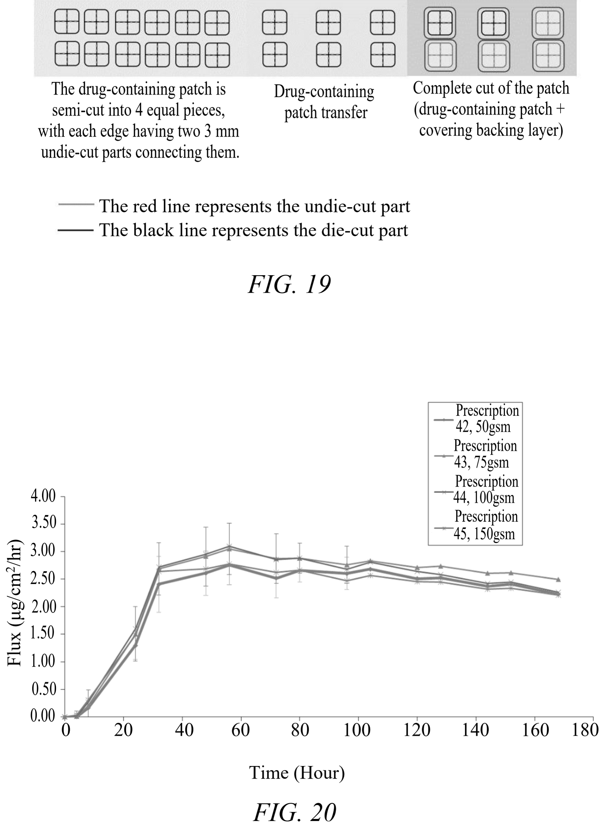

As shown in Fig. 19, the separation layer and drug matrix layer are cut into 4 small pieces of 12.5 cm$^2$ on a die-cutting machine. there are connection points between small pieces (grey); the intersecting black lines are the cut separation layer and drug matrix layer. When the inner patch (separation layer and matrix layer) moves forward, the 4 small parts of the inner patch are connected together in order.

**Example 42 to example 45** Skin flux test of patch

The transdermal patch of Examples 42-45 has the structure shown in Fig. 4B, in which the drug matrix was prepared using prescription 42 to prescription 45 in Table 11, risperidone was micronized risperidone crystal form A, and the first overlapping adhesive film was acrylic adhesive Duro-Tak 87-2054 at 55GSM, the second overlapping adhesive film was polyisobutylene at 55GSM, the separation layer was Scotchpak 1109, and the backing layer was KOB053. The matrix layer and separation layer after film-cut had connection points as shown in Figs. 16 and 19.

Table 11. Skin flux of example 42 to example 45

| time (hours) | Average value of skin from three donors | | | |
|---|---|---|---|---|
| | Example 42 | Example 43 | Example 44 | Example 45 |
| | Prescription 42 | Prescription 43 | Prescription 44 | Prescription 45 |
| | 11% risperidone, 4.12% lauryl lactate, 10% oleyl alcohol, 2.5% EPO, 0.1% DL-alpha tocopherol, 0.1% BHT, 72.18% Duro-Tak 387-2287, 50gsm | 11% risperidone, 4.12% lauryl lactate, 10% oleyl alcohol, 2.5% EPO, 0.1% DL-alpha tocopherol, 0.1% BHT, 72.18% Duro-Tak 387-2287, 75gsm | 11% risperidone, 4.12% lauryl lactate, 10% oleyl alcohol, 2.5% EPO, 0.1% DL-alpha tocopherol, 0.1% BHT, 72.18% Duro-Tak 387-2287, 100gsm | 11% risperidone, 4.12% lauryl lactate, 10% oleyl alcohol, 2.5% EPO, 0.1% DL-alpha tocopherol, 0.1% BHT, 72.18% Duro-Tak 387-2287, 150gsm |
| 0 | 0.000 | 0.000 | 0.000 | 0.000 |
| 4 | 0.000 | 0.012 | 0.010 | 0.033 |
| 8 | 0.162 | 0.200 | 0.266 | 0.303 |
| 24 | 1.296 | 1.508 | 1.598 | 1.490 |
| 32 | 2.406 | 2.685 | 2.718 | 2.641 |
| 48 | 2.605 | 2.910 | 2.949 | 2.685 |
| 56 | 2.756 | 3.047 | 3.098 | 2.773 |
| 72 | 2.514 | 2.872 | 2.858 | 2.622 |
| 80 | 2.657 | 2.880 | 2.881 | 2.659 |
| 96 | 2.599 | 2.758 | 2.674 | 2.466 |
| 104 | 2.678 | 2.826 | 2.807 | 2.565 |
| 120 | 2.507 | 2.711 | 2.635 | 2.453 |
| 128 | 2.522 | 2.733 | 2.582 | 2.448 |
| 144 | 2.369 | 2.603 | 2.417 | 2.316 |
| 152 | 2.412 | 2.611 | 2.443 | 2.330 |
| 168 | 2.226 | 2.489 | 2.262 | 2.215 |
| Average flux | 2.114 | 2.323 | 2.280 | 2.133 |

(continued)

| time (hours) | Average value of skin from three donors | | | |
|---|---|---|---|---|
| | **Example 42** | **Example 43** | **Example 44** | **Example 45** |
| | **Prescription 42** | **Prescription 43** | **Prescription 44** | **Prescription 45** |
| | 11% risperidone, 4.12% lauryl lactate, 10% oleyl alcohol, 2.5% EPO, 0.1% DL-alpha tocopherol, 0.1% BHT, 72.18% Duro-Tak 387-2287, 50gsm | 11% risperidone, 4.12% lauryl lactate, 10% oleyl alcohol, 2.5% EPO, 0.1% DL-alpha tocopherol, 0.1% BHT, 72.18% Duro-Tak 387-2287, 75gsm | 11% risperidone, 4.12% lauryl lactate, 10% oleyl alcohol, 2.5% EPO, 0.1% DL-alpha tocopherol, 0.1% BHT, 72.18% Duro-Tak 387-2287, 100gsm | 11% risperidone, 4.12% lauryl lactate, 10% oleyl alcohol, 2.5% EPO, 0.1% DL-alpha tocopherol, 0.1% BHT, 72.18% Duro-Tak 387-2287, 150gsm |
| **Decline %** | 23.290 | 18.330 | 26.990 | 20.130 |
| Risperidone = risperidone, Povidone K90 = povidone K90, Oleic acid = oleic acid, Eudragit EPO, DL-alpha tocopherol = DL-$\alpha$ tocopherol, Ascorbyl palmitate NF = ascorbyl palmitate NF, BHT = butylated hydroxytoluene, Crospovidone CL-M = crospovidone CL-M, Duro-Tak 387-2516 gsm     =     grams      per      square      meter $$\text{Decline \% = (Cmax-C168 h)/Cmax*100}$$ | | | | |

**Claims**

1. A risperidone transdermal administration system, **characterized in that** the risperidone transdermal administration system comprises:

   1) a backing layer;
   2) a matrix layer, which contains risperidone dispersed in the matrix layer in crystalline and amorphous states, a skin penetration enhancer and a pressure-sensitive adhesive; and
   3) a release layer.

2. The risperidone transdermal administration system of claim 1, **characterized in that** the crystalline form of the risperidone is crystalline form A, preferably micronized risperidone crystalline form A.

3. The risperidone transdermal administration system of claim 1 or 2, **characterized in that** the skin penetration enhancer is selected from one or more of $C_1$-$C_{30}$ fatty acids, fatty esters, or fatty alcohols;

   preferably, the fatty acids are selected from $C_{12}$-$C_{22}$ unsaturated fatty acids, more preferably oleic acid;
   preferably, the fatty esters are selected from esters produced by $C_1$-$C_{30}$ fatty acids and $C_1$-$C_{30}$ alcohols in combination, more preferably lauryl lactate,
   preferably, the fatty alcohols are selected from $C_{13}$-$C_{30}$ alkenyl alcohols, more preferably oleyl alcohol.

4. The risperidone transdermal administration system of any one of claims 1-3, **characterized in that** the matrix layer includes the following components relative to the total weight of the matrix layer:

   1) risperidone at a total amount of 9.5-15%, preferably 9.5-15% or 10-12%;
   2) the skin penetration enhancer at an amount of 1%-50%, preferably 2-30%, 8.5-30%, 8.5-25%, or 8.5-15%; more preferably, the $C_1$-$C_{30}$ fatty acids at an amount of 8.5-15%, the fatty esters at an amount of 1-10%, and the fatty alcohols at an amount of 1-15%; and most preferably, the oleic acid at an amount of 8.5-15%, the lauryl lactate at an amount of 1-10%, 1-6% or 3-5%, and the $C_{13}$-$C_{30}$ alkenyl alcohols at an amount of 1-15%, 5-15% or 8-15%;
   3) the pressure-sensitive adhesive at an amount of 50-82%, preferably 50-80% or 55-80%;

   with the total amount of all component in the matrix layer being 100%.

5. The risperidone transdermal administration system of any one of claims 1-4, **characterized in that** the matrix layer consists of the following components relative to the total weight of the matrix layer:

   1) risperidone at a total amount of 9.5-15%, preferably 9.5-15% or 10-12%;
   2) the skin penetration enhancer at an amount of 1-50%, preferably 2-30%, 8.5-30%, 8.5-25%, 8.5-15%; more preferably, the $C_1$-$C_{30}$ fatty acids at an amount of 8.5-15%, the fatty esters at an amount of 1-10%, and the fatty alcohols at an amount of 1-15%; and most preferably, oleic acid at an amount of 8.5-15%, lauryl lactate at an amount of 1-10%, 1-6% or 3-5%, $C_{13}$-$C_{30}$ alkenyl alcohol at an amount of 1-15%, 5-15% or 8-15%;
   3) the pressure-sensitive adhesive at an amount of 50-82%, preferably 50-80% or 55-80%;
   4) an antioxidant at an amount of 0%-1%, preferably 0.05%-0.5% or 0.1%-0.3%;
   5) a cohesion promoter at an amount of 0-30%, preferably 1-5%;
   6) a tackifier at an amount of 0-40%;
   7) a plasticizer at an amount of 0-40%;
   8) an additional penetration enhancer at an amount of 0-10%;

   the total amount of all component in the matrix layer is 100%.

6. The risperidone transdermal administration system of claim 5, **characterized in that** the pressure-sensitive adhesive is selected from one or more of acrylic adhesives, organosilicon adhesives, acrylic-organosilicon copolymer adhesives, polybutylene adhesives, styrene-isoprene-butylene copolymers, and styrene-butadiene-styrene copolymers.

7. The risperidone transdermal administration system of claim 5, **characterized in that** the cohesion promoter is selected from crospovidone, Eudragit E, Eudragit EPO, Eudragit S, Eudragit R. Plastoid B and mixtures thereof.

8. The risperidone transdermal administration system of claim 5, **characterized in that** the cohesion promotor is povidone K90, Eudragit EPO, and combinations of povidone K90 and Eudragit EPO, crospovidone CL-M and Eudragit EPO, povidone K90 and crospovidone CL-M and Eudragit EPO, povidone K90 and Plastoid B, crospovidone CL-M and Plastoid B, povidone K90 and crospovidone CL-M, and a preferred combination is povidone K90 and Eudragit EPO.

9. The risperidone transdermal administration system of any one of claims 5-8, **characterized in that** the antioxidant is selected from one or more of tocopherol, tocopheryl acetate, potassium metabisulfite, sodium metabisulfite, sodium bisulfite, sodium sulfite, propyl gallate, thioglycerol, sodium thiosulfate, sodium dioxide, sodium formaldehyde sulfoxylate, and butylated hydroxytoluene (BHT); and further preferably, a chelating agent as a synergistic antioxidant selecting from citric acid, tartaric acid, calcium disodium edetate, disodium edetate, and EDTA.

10. The risperidone transdermal administration system of any one of claims 5-9, **characterized in that** the tackifier is selected from polybutenes, terpenes, and mixtures thereof.

11. The risperidone transdermal administration system of any one of claims 5-10, **characterized in that** the plasticizer is selected from mineral oil, silicone oil, triethyl citrate, and mixtures thereof.

12. The risperidone transdermal administration system of any one of claims 1-11, **characterized in that** the amount of the matrix layer is from 30 g/m$^2$ to 700 g/m$^2$.

13. The risperidone transdermal administration system of any one of claims 1-12, **characterized in that** during the preparation process, risperidone is added in the form of micronized risperidone crystal form A, and preferably the particle size of the micronized risperidone is less than or equal to 20 $\mu$m.

14. The risperidone transdermal administration system of any one of claims 1-13, **characterized in that** a solubilizer or a solvent is further used in the preparation of the matrix layer, and is selected from $C_1$-$C_6$ alkyl alcohol, n-heptane, ethyl acetate, toluene and mixtures thereof; the solubilizer or the solvent is preferably ethanol, isopropanol, n-heptane or ethyl acetate.

15. The risperidone transdermal administration system of any one of claims 1-14, **characterized in that** the risperidone transdermal administration system comprises:

    1) a backing layer;
    2) a matrix layer, which contains risperidone dispersed in the matrix layer in crystalline and amorphous states, a skin penetration enhancer and a pressure-sensitive adhesive;
    3) a skin-contacting adhesive layer; and
    4) a release layer.

16. The risperidone transdermal administration system of any one of claims 1-14, **characterized in that** the risperidone transdermal administration system comprises:

    1) a backing layer;
    2) a matrix layer, which contains risperidone dispersed in the matrix layer in crystalline and amorphous states, a skin penetration enhancer and a pressure-sensitive adhesive;
    3) a semipermeable membrane or machine-woven fabric layer;
    4) a skin-contacting adhesive layer; and
    5) a release layer.

17. The risperidone transdermal administration system of claim 16 or 17, **characterized in that** the skin-contacting adhesive layer includes a pressure-sensitive adhesive and optionally risperidone and optionally other excipients selected from one or more of a cohesion promoter, an antioxidant, an anti-skin irritation agent, a tackifier, a plasticizer, a solubilizer, a solvent and a desiccant.

18. The risperidone transdermal administration system of any one of claims 1-14, **characterized in that** the risperidone transdermal administration system comprises:

    1) a backing layer;

2) an overlapping adhesive film layer, preferably the material of the overlapping adhesive film layer is selected from one or more of siloxane, polyisobutylene, or styrene-isoprene-styrene copolymer; preferably, the amount of the overlapping adhesive film layer is 50-110 GSM;

3) a separation layer;

4) a matrix layer, which contains risperidone dispersed in the matrix layer in crystalline and amorphous states, a skin penetration enhancer and a pressure-sensitive adhesive; and

5) a release layer.

19. The risperidone transdermal administration system of claim 18, **characterized in that** the overlapping adhesive film layer includes an adhesive, and the adhesive of the overlapping adhesive film layer is the same as or different from the adhesive of the drug matrix layer; preferably, the solubility of a drug in the adhesive of the overlapping adhesive film layer is the same as or less than that of the drug in the adhesive of the matrix layer; more preferably, the solubility of the drug in the adhesive of the overlapping adhesive film layer is less than that of the drug in the adhesive of the matrix layer.

20. The risperidone transdermal administration system of claim 18 or 19, **characterized in that** the overlapping adhesive film layer can be one layer, two layers or multiple layers; preferably, the first overlapping adhesive film layer close to the backing layer can prevent other overlapping adhesive film layers away from the backing layer from migrating to the backing layer or the side of the backing layer away from the skin.

21. The risperidone transdermal administration system of claim 20, **characterized in that** the adhesive of the first overlapping adhesive film layer is selected from Duro-Tak 387-2516, Duro-Tak 387-2287, Duro-Tak 387-4287, Duro-Tak 87-2051, Duro-Tak 87 -2052, Duro-Tak 87-2054, Duro-Tak 87-2852 or mixtures thereof; the adhesive of the second overlapping adhesive film layer is selected from polyisobutylene, styrene-isoprene-styrene copolymer, dimethylsiloxane, or mixtures thereof.

22. The risperidone transdermal administration system of any one of claims 18-21, **characterized in that** the risperidone transdermal administration system, from a backing layer to a disjuncting layer, includes: a backing layer, one or more overlapping adhesive film layers, a separation layer, a matrix layer, and a release layer; the backing layer, the one or more overlapping adhesive film layers and the release layer extend all around beyond the separation layer and the matrix layer; during application, the disjuncting layer is removed and the matrix layer in contact with the skin is sealed to the skin by one or more overlapping adhesive film layers.

23. The risperidone transdermal administration system of claim 22, **characterized in that** the overlapping adhesive film layer is one layer, and the risperidone transdermal administration system, from a backing layer to a disjuncting layer, includes:

   i. a backing layer, an overlapping adhesive film layer, a separation layer, a matrix layer, and a release layer, and the backing layer, the overlapping adhesive film layer, and the release layer extend all around beyond the separation layer and the matrix layer; the structure illustrated in Fig. 4A is preferred; or

   ii. a backing layer, an overlapping adhesive film layer, a separation layer, a matrix layer, a skin adhesive layer, and a release layer, and the backing layer, the overlapping adhesive film layer and the release layer extend all around beyond the separation layer, the matrix layer and the skin adhesive layer; the structure illustrated in Fig. 4C is preferred; or

   iii. a backing layer, an overlapping adhesive film layer, a separation layer, a matrix layer, a semipermeable membrane layer, a skin adhesive layer, and a release layer; and the backing layer, the overlapping adhesive film layer and the release layer extend all around beyond the separation layer, the matrix layer, the semipermeable membrane layer and skin adhesive layer; the structure illustrated in Fig. 4F is preferred.

24. The risperidone transdermal administration system of claim 22, **characterized in that** the overlapping adhesive film layer is two layers; during application, the disjuncting layer is removed and the matrix layer in contact with the skin is sealed to the skin by the second overlapping adhesive film layer; the risperidone transdermal administration system, from a backing layer to a disjuncting layer, includes:

   i. a backing layer, a first overlapping adhesive film layer, a second overlapping adhesive film layer, a separation layer, a matrix layer, and a release layer, and the backing layer, the first overlapping adhesive film layer, the second overlapping adhesive film layer and the release layer extend all around beyond the separation layer and the matrix layer; the structure illustrated in Fig. 4B is preferred; or

ii. a backing layer, a first overlapping adhesive film layer, a separation layer, a matrix layer, a skin adhesive layer, a release layer, and a backing layer, and the first overlapping adhesive film layer and the release layer extend all around beyond the separation layer, the matrix layer and the skin adhesive layer, the second overlapping adhesive film layer is filled between the first overlapping adhesive film layer and the release layer, the structure illustrated in Fig. 4D is preferred; or

iii. a backing layer, a first overlapping adhesive film layer, a separation layer, a matrix layer, a semipermeable membrane layer, a skin adhesive layer, a second overlapping adhesive film layer, and a release layer, and the backing layer, the first overlapping adhesive film layer, the second overlapping adhesive film layer and the release layer extend all around beyond the separation layer, the matrix layer, the semipermeable membrane layer and the skin adhesive layer, and the second overlapping adhesive film layer extends toward the first overlapping adhesive film layer and wraps the separation layer, the matrix layer, the semipermeable membrane layer and the skin adhesive layer; the structure illustrated in Fig. 4E is preferred;

preferably, the amount of the first overlapping adhesive film layer is 25-60 GSM; the amount of the second overlapping adhesive film layer is 25-60 GSM.

25. The risperidone transdermal administration system of any one of claims 18-24, **characterized in that** the backing layer is composed of a polymer elastic film, a polymer fabric, a bidirectional or multi-directional elastic nonwoven fabric, a stretchable polymer film, a stretchable machine-woven fabric, or a stretchable nonwoven fabric.

26. The risperidone transdermal administration system of claim 25, **characterized in that** the backing layer is selected from polyester, polyethylene, polypropylene, polyvinyl chloride, polyethylene vinyl acetate, or polyurethane, preferably KOB051 and KOB053.

27. The risperidone transdermal administration system of any one of claims 18-26, **characterized in that** the separation layer is selected from a flexible occlusive single-layer or multi-layer polymeric film, the polymer is selected from polyolefins, polyesters, polyethylene, polyvinylidene chloride or polyurethane; preferably, the separation layer further includes an aluminum film.

28. The risperidone transdermal administration system of any one of claims 18-27, **characterized in that** the separation layer includes a plurality of small pieces that are partially connected to each other, and the matrix layer includes a plurality of small pieces that are fully or partially connected;
preferably, the plurality of separation layer small pieces connected to each other in the separation layer are symmetrical or asymmetrical, the same or different, the plurality of small pieces connected to each other in the matrix layer are symmetrical or asymmetrical, the same or different; further preferably, a part of the connection points of the separation layer and a part of the connection points of the matrix layer are all aligned or partially aligned.

29. The risperidone transdermal administration system of claim 28, **characterized in that** there are one or more connection points between the adjacent small pieces, preferably 1, 2, 3, 4 or 5 connection points; and the length of each connection point is independently selected from the range of 0.5-3 mm.

30. The risperidone transdermal administration system of any one of claims 28-29, **characterized in that** overlapping separation layer pieces and matrix layer pieces have the same shape, and the number of the pieces in each transdermal administration system unit is selected from 1 to 10, each small piece has an area of 1-25 cm$^2$, preferably 1 cm$^2$, 4 cm$^2$, 6 cm$^2$, 8 cm$^2$, 9 cm$^2$, 10 cm$^2$, 12.5 cm$^2$, 16 cm$^2$, or 25 cm$^2$.

31. The risperidone transdermal administration system of any one of claims 1-30, **characterized in that** the matrix layer has a weight of 30 GSM to 700 GSM, 50 GSM to 700 GSM, 30 GSM to 100 GSM, 40 GSM to 150 GSM, 75 GSM to 150 GSM, 150 GSM to 300 GSM, or 350 GSM to 700 GSM.

32. A method for preparing the stable matrix-type risperidone transdermal administration system of any one of claims 1-14, **characterized in that** the method comprises the following steps:

step 1. mixing a skin penetration enhancer and optionally a cohesion promoter, an antioxidant, an anti-skin irritation agent, a tackifier, a plasticizer, a solubilizer, a solvent and a desiccant to obtain blend 1;
step 2. mixing the blend 1 and a pressure-sensitive adhesive to obtain blend 2;
step 3. adding micronized risperidone crystal form A or a pharmaceutically acceptable salt thereof to the blend 2 from step 2, and stirring the resulting mixture until risperidone or the pharmaceutically acceptable salt thereof is evenly suspended to obtain a wet drug mixture;

step 4. coating the wet drug mixture on a release layer;

step 5. drying to remove the solvent and the solubilizer to obtain a release layer/drug matrix layer laminated material; and

step 6. laminating the release layer/drug matrix layer laminated material to the backing layer to form a release layer/drug matrix layer/backing layer composite film.

33. The method of claim 32, **characterized in that** the method further comprises the following steps:

step 1. preparing a release layer/matrix layer/backing layer composite film according to steps 1 to 6 of the method of claim 32;

step 2. preparing a solution or a suspension of a skin-contacting adhesive layer containing a pressure-sensitive adhesive and optionally risperidone crystal form A or a pharmaceutically acceptable salt thereof and optionally other excipients, coating same to a release layer and drying to form a release layer/skin-contacting adhesive layer laminated material; and

step 3. removing the release layer from the release layer/matrix layer/backing layer composite film, and laminating the adhesive layer side of the release layer/skin-contacting adhesive layer of step 1 to the matrix layer to form a release layer/skin-contacting adhesive layer/matrix layer/backing layer composite film.

34. The method of claim 32, **characterized in that** the method further comprises the following steps:

step 1. preparing a release layer/matrix layer/backing layer composite film according to steps 1 to 6 of the method of claim 32;

step 2. preparing a solution or a suspension of a skin-contacting adhesive layer containing a pressure-sensitive adhesive and optionally risperidone crystal form A or a pharmaceutically acceptable salt thereof and optionally other excipients, coating same to a release layer and drying to form a release layer/skin-contacting adhesive layer laminated material, and laminating the adhesive layer side to a semipermeable membrane or machine-woven fabric layer; and

step 3. removing the release layer from the release layer/matrix layer/backing layer composite film, and laminating the semipermeable membrane or machine-woven fabric layer side to the matrix layer to form a release layer/skin-contacting adhesive layer/semipermeable membrane or machine-woven fabric layer/matrix layer/backing layer composite film.

35. A method for preparing the risperidone transdermal administration system of any one of claims 18-23 or 25-31, **characterized in that** the method comprises the following steps:

step A: coating the adhesive of the overlapping adhesive film layer on the release film, and after drying, compounding same to the backing layer to obtain a backing layer/overlapping adhesive film/release film composite;

step B: preparing the release layer/drug matrix layer laminated material according to steps 1 to 5 of claim 32, and laminating the release layer/drug matrix layer laminated material to the separation layer to form a release layer/drug matrix layer/separation layer film;

step C: removing the release film of the backing layer/overlapping adhesive film/release film composite of step A, and applying the separation layer of the release layer/drug matrix layer/separation layer film of step B to the overlapping adhesive film;

step D: removing the release film from the drug matrix layer and applying an oversized release film on the drug matrix layer and the overlapping adhesive film layer;

step E: die-cuting the final patch so that the overlapping adhesive film layer extends beyond the separation layer and the drug adhesive layer in each direction.

36. A method for preparing the risperidone transdermal administration system of claim 23, **characterized in that** the method comprises the following steps

step A: coating the adhesive of the overlapping adhesive film layer on the release film, and after drying, compounding same to the backing layer to obtain a backing layer/overlapping adhesive film/release film composite;

step B: preparing a release layer/skin-contacting adhesive layer/drug matrix layer drug/separation layer composite film;

step C: removing the release film of the separation layer/overlapping adhesive film/release film composite of step A, and applying the separation layer of the release layer/skin-contacting adhesive layer/matrix layer/separation layer composite film formed in step B to the overlapping adhesive film;

step D: removing the release film from the skin-contacting adhesive layer and applying an oversized release film to the skin-contacting adhesive layer and the overlapping adhesive film layer;

step E: die-cutting the final patch so that the overlapping adhesive film layer extends beyond the separation layer, the matrix layer and the skin adhesive layer in each direction; preferably, the structure of Fig. 4C is formed.

37. A method for preparing the risperidone transdermal administration system of claim 23, **characterized in that** the method comprises the following steps

step A: coating the adhesive of the overlapping adhesive film layer on the release film, and after drying, compounding same to the backing layer to obtain a backing layer/overlapping adhesive film/release film composite;

step B: preparing the release layer/skin-contacting adhesive layer/semipermeable membrane or machine-woven fabric layer/drug matrix layer/separation layer composite film;

step C: removing the release film of the backing layer/overlapping adhesive film/release film composite of step A, and applying the separation layer of the release layer/skin-contacting adhesive layer/semipermeable membrane or machine-woven fabric layer/drug matrix layer/separation layer composite film of step B to the overlapping adhesive film;

step D: removing the release film from the skin-contacting adhesive layer and applying an oversized release film to the skin-contacting adhesive layer and the overlapping adhesive film layer;

step E: die-cutting the final patch so that the backing layer, the overlapping adhesive film layer and the release layer extend all around beyond the separation layer, the matrix layer, the semipermeable membrane or machine-woven fabric layer and the skin adhesive layer; preferably, the structure of Fig. 4F is formed.

38. A method for preparing the risperidone transdermal administration system of claim 24, **characterized in that** the method comprises the following steps:

step A: coating the adhesive of the first overlapping adhesive film layer on the release film, and after drying, compounding same to the backing layer to obtain a backing layer/first overlapping adhesive film/release film composite film; coating the adhesive of the second overlapping adhesive film layer on the release film, and after drying, compounding same to the first overlapping adhesive film layer from which the release film is removed to obtain a backing layer/first overlapping adhesive film/second overlapping adhesive film/release film composite film;

step B: preparing a release layer/drug matrix layer/separation layer film, a release layer/skin adhesive layer/drug matrix layer/separation layer film, or a release layer/skin adhesive layer/semipermeable membrane machine-woven fabric layer/drug matrix layer/separation layer film;

step C: removing the release film of the backing layer/first overlapping adhesive film/second overlapping adhesive film/release film composite film of step A, and applying the separation layer of step B to the second overlapping adhesive film layer;

step D: removing the remaining release film of step C and applying an oversized release film on the side away from the backing layer;

step E: die-cutting the final patch so that the second overlapping adhesive film layer extends beyond the separation layer, the drug matrix layer, and optionally the skin adhesive layer and the semipermeable membrane machine-woven fabric layer in each direction; preferably, the structures of Figs. 4B, 4D, and 4E are obtained.

39. A method for preparing the risperidone transdermal administration system of any one of claims 28-32, **characterized in that**

after die-cutting the composite film prepared in step B of claims 35-38 into a plurality of small pieces that are partially connected, and the structure of the small pieces of claims 28-31 is obtained; the structure in step C improves the arrangement order of the composite film in step B during transfer.

40. The method of any one of claims 32-39, **characterized in that** the wet drug mixture used to prepare the matrix layer contains undissolved risperidone crystal form A.

41. The method of any one of claims 32-40, **characterized in that** the solvent in step 1 or step B is selected from $C_1$-$C_6$ alkyl alcohol, n-heptane, ethyl acetate, toluene, and mixtures thereof; preferably, ethanol, isopropanol, n-heptane or ethyl acetate.

42. A therapeutically effective amount of the risperidone transdermal administration system of any one of claims 1-41 for use in the preparation of a drug for treating or preventing schizophrenia, mania and dementia.

43. A therapeutically effective amount of the risperidone transdermal administration system of any one of claims 1-41 for use in the preparation of a drug for treating or preventing positive and negative symptoms of schizophrenia.

44. The use according to claim 43, **characterized in that** the positive and negative symptoms of schizophrenia include hallucinations, delusions, and emotional withdrawal and blunted affect.

45. A method for treating or preventing schizophrenia, mania and dementia, **characterized in that** the method comprises administering a therapeutically effective amount of the risperidone transdermal administration system of any one of claims 1-41 to a subject in need thereof.

46. A method for treating or preventing positive and negative symptoms of schizophrenia, **characterized in that** the method comprises administering a therapeutically effective amount of the risperidone transdermal administration system of any one of claims 1-41 to a subject in need thereof.

47. The method of claim 46, **characterized in that** the positive and negative symptoms of schizophrenia include hallucinations, delusions, and emotional withdrawal and blunted affect.

48. The use of any one of claims 42-44 or the method of any one of claims 45-47, **characterized in that** the risperidone transdermal administration system is administered once every 1 day, every 3 days, every 7 days, every 10 days, or every 14 days.

49. The use of any one of claims 42-44 or the method of any one of claims 45-47, **characterized in that** the risperidone transdermal administration system continuously delivers risperidone or the pharmaceutically acceptable salt thereof at a therapeutically effective amount of blood drug concentration within 24 hours to 14 days.

50. The use of any one of claims 42-44 or the method of any one of claims 45-47, **characterized in that** the risperidone transdermal administration system continuously delivers risperidone into a patient at a substantially constant rate within 24 hours to 14 days, preferably within 24 hours to 7 days.

51. The use or method of claim 50, **characterized in that** the maximum transdermal amount of risperidone occurs within 24-36 hours after administration of the risperidone transdermal administration system, and during day 3-day 7 after the administration, the transdermal amount of risperidone is maintained more than 65% of the maximum transdermal amount, preferably, maintained 65%-90% of the maximum transdermal amount of risperidone, more preferably, maintained 75%-85% of the maximum transdermal amount of risperidone.

Backing layer

Drug-adhesive layer

Release layer

*FIG. 1*

Backing layer

Matrix reservoir layer

Skin-contacting adhesive layer

Release layer

*FIG. 2*

Backing layer

Matrix reservoir layer

Membrane or machine-woven fabric

Skin-contacting adhesive layer

Release layer

*FIG. 3*

*FIG. 4A*

1. Backing layer

2.2 The second overlapping adhesive film layer

2.1 The first overlapping adhesive film layer

3. Separation layer

4. Drug matrix/skin adhesive layer

7. Disjuncting release film

*FIG. 4B*

*FIG. 4C*

*FIG. 4D*

*FIG. 4E*

*FIG. 4F*

*FIG. 5*

*FIG. 6*

*FIG. 7*

*FIG. 8*

*FIG. 9*

*FIG. 10*

*FIG. 11*

*FIG. 12*

*FIG. 13*

*FIG. 14*

The overlapping adhesive film layer is on top

The separation layer is under the overlapping adhesive film layer

*FIG. 15*

The overlapping adhesive film layer is on top

The separation layer is under the overlapping adhesive film layer

*FIG. 16*

*FIG. 17*

| The drug-containing patch is semi-cut into 4 equal pieces | The order of the four areas is changed during drug-containing patch transfer | Complete cut of the patch (drug-containing patch + covering backing layer) |

——— The black line represents the die-cut part

*FIG. 18*

The drug-containing patch is semi-cut into 4 equal pieces, with each edge having two 3 mm undie-cut parts connecting them.

Drug-containing patch transfer

Complete cut of the patch (drug-containing patch + covering backing layer)

——— The red line represents the undie-cut part
——— The black line represents the die-cut part

*FIG. 19*

Time (Hour)

*FIG. 20*

## INTERNATIONAL SEARCH REPORT

| International application No. |
|---|
| **PCT/CN2024/070458** |

| A. | CLASSIFICATION OF SUBJECT MATTER |
|---|---|

A61K31/519(2006.01)i; A61K9/70(2006.01)i; A61P25/18(2006.01)i

According to International Patent Classification (IPC) or to both national classification and IPC

| B. | FIELDS SEARCHED |
|---|---|

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; STNext; 读秀, DUXIU: 利培酮, 瑞司哌酮, 维思通, 利司培酮, 利螺环酮, 利哌酮, 瑞斯哌东, 利哌利酮, 理思必妥, 斯普里酮, 油酸, 油醇, 9-十八烯-1-醇, 顺式-9-十八碳烯醇, 乳酸月桂酯, 乳酸月桂醇酯, 乳酸月桂基酯, 月桂乳酸酯, 月桂基乳酸酯, 月桂醇乳酸酯, 贴, 透皮, 晶型, 结晶, 不溶解, 晶体, Lauryl lactate, Ceraphyl 31, Crodamol, Cyclochem, Dodecyl lactate, Lactic acid, dodecyl ester, Ocenol, HD Eutanol, Apexidone, Psychodal, R 64766, Ripedon, Rispadal, Risperdal, Risperdal Consta, Risperidal, Risperidone, Spiron, respiridone, Risoeridone, oleic acid, Patch, transdermal, insoluble, crystalline

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 101366705 A (ZHEJIANG UNIVERSITY) 18 February 2009 (2009-02-18) description, page 2, paragraphs 3 and 4, and embodiment 4 | 1, 3-12, 14-31, 36-39, 41-44, 48-51 (in part) |
| X | TW 201900177 A (TEIKOKU SEIYAKU CO., LTD.) 01 January 2019 (2019-01-01) claims 1-16 | 1, 3-12, 14-31, 36-39, 41-44, 48-51 (in part) |
| X | US 2007134310 A1 (NEDBERGE DIANE E. et al.) 14 June 2007 (2007-06-14) claims 1-45 | 1, 3-12, 14-31, 36-39, 41-44, 48-51 (in part) |
| X | US 2009181954 A1 (SHARMA SANJAY et al.) 16 July 2009 (2009-07-16) description, paragraphs [0002]-[0007] | 1, 3-12, 14-31, 36-39, 41-44, 48-51 (in part) |
| X | US 2012052112 A1 (KURIBAYASHI MITSURU et al.) 01 March 2012 (2012-03-01) description, embodiments 1-4 | 1, 3-12, 14-31, 36-39, 41-44, 48-51 (in part) |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **28 March 2024** | **11 April 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2024/070458** |

### C. DOCUMENTS CONSIDERED TO BE RELEVANT

| Category* | Citation of document, with indication, where appropriate, of the relevant passages | Relevant to claim No. |
|---|---|---|
| X | US 2014303189 A1 (GOTO MASAOKI et al.) 09 October 2014 (2014-10-09)<br>claims 1-17 | 1, 3-12, 14-31, 36-39, 41-44, 48-51 (in part) |
| X | WO 9631201 A1 (JANSSEN PHARMACEUTICA NV et al.) 10 October 1996 (1996-10-10)<br>claims 1-19 | 1, 3-12, 14-31, 36-39, 41-44, 48-51 (in part) |
| X | 齐鹏等 (QI, Peng et al.). "利培酮经皮给药系统的研制 (Study on Transdermal Delivery System of Risperdione)"<br>华西药学杂志 (*West China Journal of Pharmaceutical Sciences*),<br>Vol. 26, No. (2), 31 December 2011 (2011-12-31), pp. 112-114<br>sections 1.2.2, 1.2.4 and 1.2.5 | 1, 3-12, 14-31, 36-39, 41-44, 48-51 (in part) |
| Y | CN 101366705 A (ZHEJIANG UNIVERSITY) 18 February 2009 (2009-02-18)<br>description, page 2, and embodiment 4 | 2, 13, 32-35, 40 |
| Y | WO 2008044336 A1 (HISAMITSU PHARMACEUTICAL CO. et al.) 17 April 2008 (2008-04-17)<br>description, paragraphs [0006] and [0008] | 2, 13, 32-35, 40 |
| Y | US 2002004063 A1 (ZARS INC.) 10 January 2002 (2002-01-10)<br>description, paragraphs [0004] and [0083] | 2, 13, 32-35, 40 |
| A | CN 110038130 A (ZHANG, Jie) 23 July 2019 (2019-07-23)<br>claims 1-12 | 1-44, 48-51 (in part) |

Form PCT/ISA/210 (second sheet) (July 2022)

## INTERNATIONAL SEARCH REPORT

| International application No. |
| --- |
| **PCT/CN2024/070458** |

**Box No. II      Observations where certain claims were found unsearchable (Continuation of item 2 of first sheet)**

This international search report has not been established in respect of certain claims under Article 17(2)(a) for the following reasons:

1. ☑   Claims Nos.: **45-47, 48-51 (in part)**
because they relate to subject matter not required to be searched by this Authority, namely:

The method involved in claims 45-47 and claims 48-51, when refer thereto, is a disease treatment method that is practiced on a living human or animal body for the direct purpose of disease treatment, falls within methods for treatment of the human or animal body by therapy, and falls within the cases set out in PCT Rule 39.1(iv) for which an international search is not required.

2. ☐   Claims Nos.:
because they relate to parts of the international application that do not comply with the prescribed requirements to such an extent that no meaningful international search can be carried out, specifically:

3. ☐   Claims Nos.:
because they are dependent claims and are not drafted in accordance with the second and third sentences of Rule 6.4(a).

**INTERNATIONAL SEARCH REPORT**
Information on patent family members

International application No.

**PCT/CN2024/070458**

| Patent document cited in search report | | | Publication date (day/month/year) | Patent family member(s) | | | Publication date (day/month/year) |
|---|---|---|---|---|---|---|---|
| CN | 101366705 | A | 18 February 2009 | None | | | |
| TW | 201900177 | A | 01 January 2019 | US | 2020108024 | A1 | 09 April 2020 |
| | | | | US | 11382870 | B2 | 12 July 2022 |
| | | | | JPWO | 2018186441 | A1 | 16 April 2020 |
| | | | | JP | 7138624 | B2 | 16 September 2022 |
| | | | | TWI | 783984 | B | 21 November 2022 |
| | | | | WO | 2018186441 | A1 | 11 October 2018 |
| US | 2007134310 | A1 | 14 June 2007 | WO | 2007035942 | A2 | 29 March 2007 |
| | | | | WO | 2007035942 | A3 | 25 October 2007 |
| US | 2009181954 | A1 | 16 July 2009 | EP | 2230911 | A1 | 29 September 2010 |
| | | | | EP | 2230911 | A4 | 03 July 2013 |
| | | | | US | 2015202207 | A1 | 23 July 2015 |
| | | | | US | 8927537 | B2 | 06 January 2015 |
| | | | | JP | 2011507904 | A | 10 March 2011 |
| | | | | WO | 2009086137 | A1 | 09 July 2009 |
| US | 2012052112 | A1 | 01 March 2012 | JPWO | 2010098261 | A1 | 30 August 2012 |
| | | | | WO | 2010098261 | A1 | 02 September 2010 |
| US | 2014303189 | A1 | 09 October 2014 | JPWO | 2013027681 | A1 | 19 March 2015 |
| | | | | WO | 2013027681 | A1 | 28 February 2013 |
| WO | 9631201 | A1 | 10 October 1996 | IL | 117809 | A0 | 04 August 1996 |
| | | | | IL | 117809 | A | 31 October 2000 |
| | | | | CY | 2388 | B1 | 10 September 2004 |
| | | | | PT | 879051 | E | 31 October 2003 |
| | | | | ZA | 9602752 | B | 06 October 1997 |
| | | | | ATE | 242631 | T1 | 15 June 2003 |
| | | | | DE | 69628672 | D1 | 17 July 2003 |
| | | | | DE | 69628672 | T2 | 29 April 2004 |
| | | | | AU | 5149296 | A | 23 October 1996 |
| | | | | AU | 696789 | B2 | 17 September 1998 |
| | | | | DK | 0879051 | T3 | 29 September 2003 |
| | | | | HK | 1015681 | A1 | 22 October 1999 |
| | | | | AR | 003413 | A1 | 05 August 1998 |
| | | | | EP | 0879051 | A1 | 25 November 1998 |
| | | | | EP | 0879051 | B1 | 11 June 2003 |
| | | | | EP | 0879051 | B9 | 03 March 2004 |
| | | | | KR | 100437672 | B1 | 10 September 2004 |
| | | | | HRP | 960154 | A2 | 31 August 1997 |
| | | | | ES | 2201176 | T3 | 16 March 2004 |
| | | | | JPH | 11503138 | A | 23 March 1999 |
| | | | | TW | 592729 | B | 21 June 2004 |
| | | | | CA | 2214420 | A1 | 10 October 1996 |
| | | | | ZA | 962752 | B | 06 October 1997 |
| WO | 2008044336 | A1 | 17 April 2008 | JPWO | 2008044336 | A1 | 04 February 2010 |
| | | | | JP | 5243254 | B2 | 24 July 2013 |
| US | 2002004063 | A1 | 10 January 2002 | PT | 1244401 | E | 28 February 2007 |
| | | | | JP | 2012246315 | A | 13 December 2012 |
| | | | | AU | 7719800 | A | 30 April 2001 |
| | | | | ES | 2276697 | T3 | 01 July 2007 |
| | | | | JP | 2003510259 | A | 18 March 2003 |

Form PCT/ISA/210 (patent family annex) (July 2022)

# EP 4 647 074 A1

## INTERNATIONAL SEARCH REPORT
### Information on patent family members

International application No.

**PCT/CN2024/070458**

| Patent document cited in search report | | | Publication date (day/month/year) | Patent family member(s) | | | Publication date (day/month/year) |
|---|---|---|---|---|---|---|---|
| | | | | EP | 1244401 | A1 | 02 October 2002 |
| | | | | EP | 1244401 | A4 | 11 August 2004 |
| | | | | EP | 1244401 | B1 | 22 November 2006 |
| | | | | EP | 1702597 | A2 | 20 September 2006 |
| | | | | EP | 1702597 | A3 | 26 March 2008 |
| | | | | JP | 2007126462 | A | 24 May 2007 |
| | | | | DK | 1244401 | T3 | 19 February 2007 |
| | | | | DE | 60032021 | D1 | 04 January 2007 |
| | | | | DE | 60032021 | T2 | 14 June 2007 |
| | | | | ATE | 345757 | T1 | 15 December 2006 |
| | | | | CA | 2386017 | A1 | 05 April 2001 |
| | | | | CA | 2386017 | C | 06 April 2010 |
| | | | | JP | 2007217431 | A | 30 August 2007 |
| | | | | WO | 0122907 | A1 | 05 April 2001 |
| | | | | US | 6528086 | B2 | 04 March 2003 |
| CN | 110038130 | A | 23 July 2019 | EP | 3741364 | A1 | 25 November 2020 |
| | | | | EP | 3741364 | A4 | 27 October 2021 |
| | | | | WO | 2019141178 | A1 | 25 July 2019 |
| | | | | US | 2021023018 | A1 | 28 January 2021 |

Form PCT/ISA/210 (patent family annex) (July 2022)

55

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4804663 A
- EP 0196132 A
- CN 101366705 B
- US 8431152 B1
- WO 0212200 A1